# EUROPÄISCHE PATENTANMELDUNG

(11) **EP 2 267 145 A1**
(43) Veröffentlichungstag der Anmeldung: **29.12.2010**
(21) Anmeldenummer: 10167125.3
(22) Anmeldetag: 24.06.2010
(51) Int. Cl.: C12P 13/04, C12P 13/08

(54) **Verfahren zur Herstellung von L-Aminosäuren unter Verwendung von verbesserten Stämmen der Familie Enterobacteriaceae**

(30) Priorität: 24.06.2009 DE 102009030193
(71) Anmelder: Evonik Degussa GmbH, 45128 Essen (DE)
(72) Erfinder: Rieping, Mechthild, 33619 Bielefeld (DE)

(57) **Zusammenfassung**

Die Erfindung betrifft ein Verfahren zur Herstellung von L-Aminosäuren durch Fermentation von rekombinanten Mikroorganismen der Familie Enterobacteriaceae, dadurch gekennzeichnet, dass man
a) die die gewünschte L-Aminosäure produzierenden Mikroorganismen, in denen man den yhaO-ORF, für das Genprodukt kodierende Nukleotidsequenzen oder Allele abschwächt, insbesondere ausschaltet, in einem Medium kultiviert unter Bedingungen, bei denen die gewünschte L-Aminosäure im Medium oder in den Zellen angereichert wird, und
b) die gewünschte L-Aminosäure isoliert, wobei gegebenenfalls Bestandteile der Fermentationsbrühe und/oder die Biomasse in ihrer Gesamtheit oder Anteilen (> 0 bis 100 %) im isolierten Produkt verbleiben oder vollständig entfernt werden.

## Beschreibung

Diese Erfindung betrifft ein Verfahren zur Herstellung von L-Aminosäuren, insbesondere L-Threonin, unter Verwendung von rekombinanten Mikroorganismen der Familie Enterobacteriaceae, in denen mindestens der offene Leserahmen (ORF) yhaO, kodierend für einen putativen Aminosäure/Proton-Symporter der HAAAP (Hydroxy/Aromatic Amino Acid Permease) Familie, abgeschwächt wird, und diese Mikroorganismen.

### Stand der Technik

L-Aminosäuren, insbesondere L-Threonin, finden in der Humanmedizin und in der pharmazeutischen Industrie, in der Lebensmittelindustrie und ganz besonders in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie z.B. die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht Aminosäure überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren.

Seit einigen Jahren werden ebenfalls Methoden der rekombinanten DNA-Technik zur gezielten Stammverbesserung L-Aminosäuren produzierender Stämme der Familie Enterobacteriaceae eingesetzt, indem man einzelne Aminosäure-Biosynthesegene amplifiziert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)).

Neben der Verstärkung des zur Herstellung der L-Aminosäure benötigten Biosyntheseweges ist auch die Abschwächung oder Ausschaltung der die L-Aminosäure-abbauenden Enzyme, eine gesteigerte Export-Rate und entsprechend eine verminderte Wiederaufnahme-Rate der L-Aminosäure von grundlegender Bedeutung für eine gesteigerte Aminosäure-Produktion (Rieping und Hermann, Microbiology Monographs, Springer-Verlag Berlin/Heidelberg, Vol. 9, 71-92 (2006)).

### Aufgabe der Erfindung

Die Erfinder haben sich die Aufgabe gestellt, neue Maßnahmen zur verbesserten fermentativen Herstellung von L-Aminosäuren, insbesondere L-Threonin, bereitzustellen.

### Beschreibung der Erfindung

Gegenstand der Erfindung ist ein Verfahren zur fermentativen Herstellung von L-Aminosäuren, insbesondere L-Threonin, unter Verwendung von rekombinanten Mikroorganismen der Familie Enterobacteriaceae, die insbesondere bereits L-Aminosäuren produzieren, und in denen mindestens der offene Leserahmen (ORF) yhaO, der für einen putativen Aminosäure/Proton-Symporter kodiert, oder für dessen Genprodukt kodierende Nukleotidsequenzen abgeschwächt, insbesondere ausgeschaltet wird bzw. werden, und die vermehrt L-Aminosäuren, insbesondere L-Threonin, produzieren und in der Zelle oder im Medium anreichern.

Als Ausgangspunkt für den Vergleich dienen jeweils die für den yhaO-ORF nicht rekombinanten Mikroorganismen, die keinen abgeschwächten yhaO-ORF enthalten und an denen die erfindungsgemäße Abschwächung oder Ausschaltung nicht durchgeführt wurde.

Bevorzugt werden die beschriebenen Mikroorganismen eingesetzt.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin gemeint. Besonders bevorzugt ist L-Threonin.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Gegenstand der Erfindung ist ein Verfahren zur Herstellung von L-Aminosäuren oder diese enthaltenden Futtermitteladditiven durch Fermentation von rekombinanten Mikroorganismen der Familie Enterobacteriaceae, **dadurch gekennzeichnet, dass** man
a) die die gewünschte L-Aminosäure produzierenden Mikroorganismen, in denen mindestens der offene Leserahmen (ORF) yhaO oder für dessen Genprodukt kodierende Nukleotidsequenzen oder Allele abschwächt, insbesondere ausschaltet, in einem Medium kultiviert unter Bedingungen, bei denen die gewünschte L-Aminosäure im Medium oder in den Zellen angereichert wird, und,
b) die gewünschte L-Aminosäure isoliert, wobei gegebenenfalls weitere Bestandteile der Fermentationsbrühe und/oder die Biomasse in ihrer Gesamtheit oder Anteilen, im isolierten Produkt verbleiben.

Die insbesondere rekombinanten Mikroorganismen mit mindestens einem abgeschwächten oder ausgeschalteten offenen Leserahmen yhaO, die ebenfalls Gegenstand der vorliegenden Erfindung sind, können L-Aminosäuren aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, die insbesondere bereits vor Durchführung der Maßnahmen dieser Erfindung L-Aminosäuren über den Eigenbedarf hinaus produzieren, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Nicht geeignet sind Wildtypstämme, in denen das genannte ORF abgeschwächt oder ausgeschaltet wird, die die gewünschte Aminosäure nur reguliert für den Eigenbedarf produzieren.

Rekombinante Mikroorganismen werden im Allgemeinen durch Transformation, Transduktion oder Konjugation, oder einer Kombination dieser Methoden, mit einem Vektor erzeugt, der den gewünschten ORF, das gewünschte Gen, ein Allel dieses ORFs oder Gens oder Teile davon und/oder einen die Exprimierung des ORFs oder Gens abschwächenden Promotor enthält. Bei diesem Promotor kann es sich um den durch abschwächende Mutation aus der eigenen, upstream des Gens oder ORFs befindlichen regulatorischen Sequenz hervorgegangenen Promotor handeln, oder es wurde ein schwacher Promotor mit dem Gen oder ORF fusioniert.

Als Elternstamm eignen sich Mikroorganismen der Familie Enterobacteriaceae, die insbesondere bereits vor Durchführung der Maßnahmen dieser Erfindung L-Aminosäuren über den Eigenbedarf hinaus produzieren und insbesondere ausscheiden.

Als zum Elternstamm geeignete, insbesondere L-Threonin produzierende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli TH 14.97 (WO 02/26993)
- Escherichia coli TH 21.97 (WO 02/26993)
- Escherichia coli BKIIM B-3996 (US-A-5,175,107)
- Escherichia coli BKIIM B-3996ΔtdhΔpckA/pVIC40 (WO 02/29080)
- Escherichia coli kat 13 (WO 98/04715)
- Escherichia coli Kat 69.9 (WO 02/26993)
- Escherichia coli KCCM-10132 (WO 00/09660)
- Escherichia coli KCCM-10168 (WO 01/14525)
- Escherichia coli KCCM-10133 (WO 00/09661)

Als zum Elternstamm geeignete L-Threonin produzierende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (AppliedBiochemistry and Biotechnology 37(3): 255-265 (1992))

L-Threonin produzierende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Es wurde gefunden, dass Mikroorganismen der Familie Enterobacteriaceae nach Abschwächung oder Ausschaltung des Gens oder offenen Leserahmens (ORF) yhaO, oder dessen Allelen, vermehrt L-Aminosäuren, insbesondere L-Threonin, produzieren und in der Zelle oder im Medium anreichern.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminopeptidase) die N-terminale Aminosäure Methionin abgespalten werden kann.

Aus den ebenfalls zur Familie Enterobacteriaceae gehörenden Salmonella typhimurium und Shigella flexneri ist die Nukleotidsequenz für den yhaO-ORF ebenso bekannt (Accession No.: NC_003197 (Region: 3403351 <- 3404691) bzw. Accession No.: NC_004337 (Region: 3248185 <- 3249516)).

Der yhaO-ORF von Escherichia coli K12 wird unter anderem durch folgende Angaben beschrieben:

Das Genprodukt des yhaO-ORFs ist annotiert als putatives Permease-Protein (Aminosäure/Proton-Symporter) eines Transportsystems der HAAAP (Hydroxy/Aromatic Amino Acid Permease) Familie mit einer Länge von 422 Aminosäuren.

Accession No.: U00096 (Region: 3254701 <- 3255969) Alternativer Genname: b3110

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum yhaO-ORF von Escherichia coli unter der SEQ ID No. 1 und die bekannten Sequenzen zum yhaO-ORF von Salmonella typhimurium und Shigella flexneri unter der SEQ ID No. 3 bzw. 5 dargestellt. Die von diesen Leserahmen kodierten Proteine sind als SEQ ID No. 2 und SEQ ID No. 4 bzw. SEQ ID No. 6 dargestellt.

Die in den angegebenen Textstellen beschriebenen offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des yhaO-ORFs, welche funktionsneutrale Sinnmutationen enthalten, zählen unter anderem solche, die zu höchstens 30 oder zu höchstens 20, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 30, 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 10 in keinem Falle aber mehr als 20 Aminosäuren betreffen.

Zu den geeigneten Allelen gehören ferner solche die durch Hybridisierung, insbesondere unter stringenten Bedingungen unter Verwendung von SEQ ID No. 1 oder SEQ ID No. 3 bzw. SEQ ID No. 5 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1 oder SEQ ID No. 3 bzw. SEQ ID No. 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001). Weiterhin kann es für die Produktion von L-Aminosäuren, insbesondere L-Threonin mit Stämmen der Familie Enterobacteriaceae vorteilhaft sein, zusätzlich zur Abschwächung des offenen Leserahmens yhaO, ein oder mehrere Enzyme des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Der hier verwendete Ausdruck "funktionell verknüpft" bedeutet, dass zwei miteinander verknüpfte Sequenzen in der beabsichtigten Weise funktionieren, dass zum Beispiel eine regulatorische Sequenz wie ein Promotor, funktionell verknüpft mit einem Gen, die Expression dieses Gens steuert.

Rekombinante Mikroorganismen mit einer solchen Verstärkung werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens oder ORFs verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Verstärkung oder Überexpression durchgeführt wird.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin- und L-Tryptophan-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die ORFs, Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, Ipp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Der Austausch des Promotors gegen einen zum Beispiel in genomweit-vergleichenden Expressionsanalysen ermittelten Promotor mit gleichmäßig hoher Expression im Verlauf eines Fermentationsprozesses bewirkt eine gleichmäßige Verstärkung. Eine andere zeitliche Genexpression kann beispielsweise wie bei Walker et al. (Journal of Bacteriology 181: 1269-80 (1999)) beschrieben durch die Verwendung des Wachstumsphasenabhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden. Desweiteren kann der Austausch eines Start-Codons zu dem in Escherichia coli mit 77% am häufigsten vorkommenden Codon ATG die Translation erheblich verbessern, da das Codon AUG zweibis dreimal effektiver ist als zum Beispiel die Codons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Codons kann optimiert werden, da zusammenwirkende Effekte zwischen dem Start-Codon und den flankierenden Bereichen beschrieben sind (Stenstrom et al., Gene 273(2):259-65 (2001); Hui et al., EMBO Journal 3(3):623-9 (1984)).

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolomé et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens eines oder mehrere der im folgenden genannten Gene oder für deren Genprodukte kodierende Nukleotidsequenzen oder Allele trägt.

So können beispielsweise gleichzeitig eines oder mehrere der Gene, ausgewählt aus der Gruppe
- mindestens ein Gen des für die Aspartatkinase, die Homoserin-Dehydrogenase, die Homoserinkinase und die Threoninsynthase kodierenden thrABC-Operons (US-A-4,278,765), gegebenenfalls der feed back resistenten Form,
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992); WO 97/08333),
- das für die Phosphoenolpyruvat-Carboxylase kodierende ppc-Gen (WO 02/064808),
- die für die Untereinheiten der Transhydrogenase kodierenden Gene pntA und pntB (European Journal of Biochemistry 158: 647-653 (1986); WO 95/11985),
- das für das Threoninresistenz vermittelnde Protein kodierende Gen rhtC
   (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983); DE19907347),
- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glucose-spezifische IIA Komponente des Phosphotransferase-Systems PTS kodierende crr-Gen des ptsHIcrr-Operons (WO 03/004674),
- das für die Glucose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Reduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD-Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615),
- das Genprodukt des offenen Leserahmens (ORF) yibD von Escherichia coli (Accession Number AE000439 des National Center for Biotechnology Information (NCBI, Bethesda, MD, USA), DE102004005836.9),
- das Genprodukt des offenen Leserahmens (ORF) yjcG von Escherichia coli (Accession Number NC000913 (Region 4281276-4282925) des National Center for Biotechnology Information (NCBI, Bethesda, MD, USA),
- das Genprodukt des offenen Leserahmens (ORF) ytfR von Escherichia coli (Accession Number NC000913 (Region 4449081-4450583) des National Center for Biotechnology Information (NCBI, Bethesda, MD, USA), der auch unter der Bezeichnung ytfS-ORF bekannt ist,
- das Genprodukt des offenen Leserahmens (ORF) ytfT von Escherichia coli (Accession Number NC000913 (Region 4450594-4451619) des National Center for Biotechnology Information (NCBI, Bethesda, MD, USA),
- das Genprodukt des offenen Leserahmens (ORF) yjfF von Escherichia coli (Accession Number NC000913 (Region 4451630-4452601) des National Center for Biotechnology Information (NCBI, Bethesda, MD, USA), und
- das Genprodukt des offenen Leserahmens (ORF) ytfQ von Escherichia coli (Accession Number NC000913 (Region 4447985-4448941) des National Center for Biotechnology Information (NCBI, Bethesda, MD, USA)
verstärkt, insbesondere überexprimiert werden.

L-Threonin produzierende Mikroorganismen der Familie Enterobacteriaceae besitzen typischerweise eine "feed back" resistente bzw. desensibilisierte Aspartatkinase I/Homoserin-Dehydrogenase I. Unter "feed back" resistenter Aspartatkinase/Homoserin-Dehydrogenase versteht man Aspartatkinase/Homoserin-Dehydrogenase Enzyme (kodiert durch thrA, EC:2.7.2.4/ EC:1.1.1.3), die im Vergleich zur Wildform eine geringere Empfindlichkeit gegenüber der Hemmung durch Threonin oder Mischungen von Threonin und dem Threonin-Analogon α-Amino-β-Hydroxyvaleriansäure (AHV) oder AHV allein aufweisen. Die für diese desensibilisierten Enzyme kodierenden Gene bzw. Allele werden auch als thrA^{FBR}-Allele bezeichnet. Im Stand der Technik sind thrA^{FBR}-Allele beschrieben, die für Aspartatkinase/Homoserin-Dehydrogenase Varianten kodieren, welche Aminosäureaustausche im Vergleich zum Wildtypprotein besitzen. Die Kodierregion des Wildtyp thrA-Gens von Escherichia coli entsprechend der Zugangsnummer U00096.2 der NCBI Datenbank (Bethesda, MD, USA) ist in SEQ ID No.7 und das von diesem Gen kodierte Polypeptid in SEQ ID No.8 dargestellt.

Auch die Nukleotidsequenz des thrA-Gens von Serratia marcescens ist bekannt und unter der Zugangsnummer X60821 beim NCBI verfügbar. Die Kodierregion des Wildtyp thrA-Gens von Serratia marcescens ist in SEQ ID No.9 und das von diesem Gen kodierte Polypeptid in SEQ ID No.10 dargestellt.

Die für die Maßnahmen der Erfindung eingesetzten L-Threonin produzierenden Mikroorganismen der Familie Enterobacteriaceae verfügen bevorzugt über ein thrA-Allel, das für eine Aspartatkinase/Homoserin-Dehydrogenase Variante kodiert, welche die Aminosäuresequenz von SEQ ID No.8 oder SEQ ID No.10 besitzt, wobei diese eine oder mehrere der Aminosäureaustausche ausgewählt aus der Gruppe:
ThrA E253K (Austausch von L-Glutaminsäure an Position 253 des kodierten Enzyms Aspartatkinase/Homoserin-Dehydrogenase gemäß SEQ ID No.8 oder SEQ ID No.10 gegen L-Lysin; siehe Research Disclosure 505, 537 (2006)),
ThrA G330D (Austausch von Glycin an Position 330 des kodierten Enzyms Aspartatkinase/Homoserin-Dehydrogenase gemäß SEQ ID No.8 oder SEQ ID No.10 gegen L-Asparaginsäure; siehe Omori et al. (Journal of Bacteriology 175(3), 785-794 (1993)),
ThrA S345F (Austausch von L-Serin an Position 345 des kodierten Enzyms Aspartatkinase/Homoserin-Dehydrogenase gemäß SEQ ID No.8 oder SEQ ID No.10 gegen L-Phenylalanin; siehe Lee et al., Journal of Bacteriology 185(18): 5442-5451 (2003)),
ThrA S352, Austausch von L-Serin an Position 352 des kodierten Enzyms Aspartatkinase/Homoserin-Dehydrogenase gemäß SEQ ID No.8 oder SEQ ID No.10 gegen L-Phenylalanin, L-Tyrosin, L-Asparagin, L-Alanin, L-Arginin, L-Glutamin, L-Glutaminsäure, L-Histidin, L-Leucin, L-Methionin, L-Tryptophan oder L-Valin, bevorzugt gegen L-Phenylalanin; siehe Omori et al. (Journal of Bacteriology 175(3), 785-794 (1993) und Omori et al. (Journal of Bacteriology 175(4), 959-965 (1993),
ThrA A479T (Austausch von L-Alanin an Position 479 des kodierten Enzyms Aspartatkinase/Homoserin-Dehydrogenase gemäß SEQ ID No.8 oder SEQ ID No.10 gegen L-Threonin; siehe Omori et al. (Journal of Bacteriology 175(3), 785-794 (1993)),
umfasst.

Bevorzugt wird eines der thrA^{FBR}-Allele thrA E253K (Austausch von L-Glutaminsäure an Position 253 des kodierten Enzyms Aspartatkinase/Homoserin-Dehydrogenase gegen L-Lysin) und S345F (Austausch von L-Serin an Position 345 des kodierten Enzyms Aspartatkinase/Homoserin-Dehydrogenase gegen L-Phenylalanin) gemäß SEQ ID No.8.

Die hier beschriebenen thrA^{FBR}-Allele, die für ein Aspartatkinase/Homoserin-Dehydrogenase Enzym kodieren, können durch gängige genetische Techniken wie ortsgerichtete Mutagenese erhalten und mit den oben beschriebenen Maßnahmen überexprimiert werden.

Gegebenenfalls kann es von Vorteil sein, nur eine gemäßigte Erhöhung der intrazellulären Aktivität oder Konzentration eines oder mehrerer Enzyme oder Proteine in einem Mikroorganismus durchzuführen, da eine zu starke Erhöhung zum Beispiel zu einer fehlerhaften Zellteilung oder veränderter Zellmorphologie bis hin zur Toxizität führen kann (Guthrie und Wickner, Journal of Bacteriology 172(10):5555-5562 (1990); Genevaux P. et al.; EMBO Reports 5(2): 195-200 (2004)).

Der Begriff "gemäßigte Erhöhung" beschreibt die Erhöhung der intrazellulären Aktivität oder Konzentration des entsprechenden Proteins um höchstens das 10-fache, das 8-fache, das 6-fache, das 4-fache, das 3-fache, das 2-fache oder das 1,5-fache bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm. Als nicht rekombinanter Mikroorganismus oder Elternstamm (parent strain) wird der Mikroorganismus verstanden, an dem die erfindungsgemäße Verstärkung oder Überexpression durchgeführt wird.

Weiterhin kann es für die Produktion von L-Aminosäuren, insbesondere L-Threonin vorteilhaft sein, zusätzlich zur Abschwächung des offenen Leserahmens yhaO, eines oder mehrere der Gene ausgewählt aus der Gruppe
- das für die Threonin-Dehydrogenase kodierende tdh-Gen (Journal of Bacteriology 169: 4716-4721 (1987)),
- das für die Malat-Dehydrogenase (E.C. 1.1.1.37) kodierende mdh-Gen (Archives in Microbiology 149: 36-42 (1987)),
- das Genprodukt des offenen Leserahmens (ORF) yjfA von Escherichia coli (Accession Number AAC77180 des National Center for Biotechnology Information (NCBI, Bethesda, MD, USA), WO 02/29080),
- das Genprodukt des offenen Leserahmens (ORF) ytfP von Escherichia coli (Accession Number AAC77179 des National Center for Biotechnology Information (NCBI, Bethesda, MD, USA), WO 02/29080),
- das für das Enzym Phosphoenolpyruvat-Carboxykinase kodierende pckA-Gen (WO 02/29080),
- das für die Pyruvat-Oxidase kodierende poxB-Gen (WO 02/36797),
- das für den DgsA-Regulator des Phosphotransferase-Systems kodierende dgsA-Gen (WO 02/081721), das auch unter der Bezeichnung mlc-Gen bekannt ist,
- das für den Fructose-Repressor kodierende fruR-Gen (WO 02/081698), das auch unter der Bezeichnung cra-Gen bekannt ist,
- das für den Sigma³⁸-Faktor kodierende rpoS-Gen (WO 01/05939), das auch unter der Bezeichnung katF-Gen bekannt ist,
- das für die Aspartat Ammonium-Lyase kodierende aspA-Gen (WO 03/008603),
- das für das periplasmatische Bindeprotein eines hochaffinen ABC-Transporters für Leucin, Isoleucin und Valin kodierende livJ-Gen,
- das für das periplasmatische Bindeprotein eines hochaffinen ABC-Transporters für Leucin, Isoleucin und Valin kodierende livK-Gen,
- das für einen Na⁺-gekoppelten Serin/Threonin-Symporter der DAACS Familie kodierende sstT-Gen, der besseren Übersichtlichkeit halber ist die Sequenz des sstT-Gens unter der SEQ ID No. 11 und die des von diesem Leserahmen kodierten Proteins als SEQ ID No. 12 dargestellt),
- das für einen Threonin/Proton-Symporter der STP Familie kodierende tdcC-Gen, der besseren Übersichtlichkeit halber ist die Sequenz des tdcC-Gens unter der SEQ ID No. 13 und die des von diesem Leserahmen kodierten Proteins als SEQ ID No. 14 dargestellt),
- das Genprodukt des offenen Leserahmens (ORF) yhjV von Escherichia coli (Accession Number NC_ 000913.2 (3,698,586 -> 3,699,857) des National Center for Biotechnology Information (NCBI, Bethesda, MD, USA)), der besseren Übersichtlichkeit halber ist die Sequenz des yhjV-ORFs unter der SEQ ID No. 15 und die des von diesem Leserahmen kodierten Proteins als SEQ ID No. 16 dargestellt), und
- das Genprodukt des offenen Leserahmens (ORF) yqeG von Escherichia coli (Accession Number NC_000913.2 (2,983,869 -> 2,985,098) des National Center for Biotechnology Information (NCBI, Bethesda, MD, USA)), der besseren Übersichtlichkeit halber ist die Sequenz des yqeG-ORFs unter der SEQ ID No. 17 und die des von diesem Leserahmen kodierten Proteins als SEQ ID No. 18 dargestellt),
abzuschwächen, insbesondere auszuschalten oder die Expression zu verringern.

Weiterhin kann es für die Produktion von L-Aminosäuren, insbesondere L-Threonin vorteilhaft sein, zusätzlich zur Abschwächung des offenen Leserahmens yhaO, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Gegebenenfalls können die Maßnahmen zur Verstärkung und zur Abschwächung beliebig kombiniert werden.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben verbessert.

Die erfindungsgemäß hergestellten Mikroorganismen können im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen.
Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung der L-Aminosäure in der Kulturbrühe. Die Kultur wird solange fortgesetzt, bis sich ein Maximum an L-Aminosäuren bzw. L-Threonin gebildet hat. Dieses Ziel wird normalerweise innerhalb von 10 Stunden bis 160 Stunden erreicht.

Aus der entnommenen Kulturbrühe können die L-Aminosäuren gewonnen, gesammelt oder konzentriert und gegebenenfalls gereinigt werden. Typische Methoden zur Reinigung der L-Aminosäuren sind die Ionenaustauschchromatographie und die Kristallisation. Hierdurch erhält man weitgehend reine L-Aminosäuren.

Es ist ebenfalls möglich aus der entnommenen Kulturbrühe (= Fermentationsbrühe) ein Produkt herzustellen, indem man die in der Kulturbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10%, kleiner als 5% oder kleiner 3% beträgt.

Die Analyse von L-Aminosäuren zur Bestimmung der Konzentration zu einem oder mehreren Zeitpunkt(en) im Verlauf der Fermentation kann durch Trennung der L-Aminosäuren mittels Anionenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Derivatisierung eingesetzt werden.

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben.

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Das erfindungsgemäße Verfahren dient zur fermentativen Herstellung von L-Aminosäuren, wie beispielsweise L-Threonin, L-Isoleucin, L-Valin, L-Methionin, L-Homoserin, L-Tryptophan und L-Lysin, insbesondere L-Threonin.

### Beispiele

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America, USA (1989) 86: 2172-2175) durchgeführt.

Die Bebrütungstemperatur bei der Herstellung von Stämmen und Transformanten ist, wenn nicht anders beschrieben, 37°C.

### Beispiel 1

### Konstruktion der Deletionsmutation des yhaO-Gens

Teile der stromaufwärts und -abwärts des yhaO-Gens liegenden Genregionen und Teile der 5'- und 3'-Region des yhaO-Gens werden aus Escherichia coli K12 unter Anwendung der "gene SOEing"-Polymerase-Kettenreaktion (PCR) (Gene Splicing by Overlap Extension, Horton, Molecular Biotechnology 3: 93-98 (1995)) sowie synthetischen Oligonukleotiden amplifiziert. Ausgehend von der Nukleotidsequenz des yhaO-Gens und stromaufwärts und - abwärts liegender Sequenzen in E. coli K12 MG1655 (SEQ ID No. 1, Accession Number U00096 (Region: 3254701 <-3255969)) werden PCR-Primer synthetisiert (MWG Biotech, Ebersberg, Deutschland). Die Sequenzen der Primer yhaO-1 und yhaO-4 wurden so modifiziert, dass Erkennungsstellen für Restriktionsenzyme entstanden. Für beide Primer wurde die Erkennungssequenz für SalI gewählt, die in der unten dargestellten Nukleotidabfolge durch Unterstreichen markiert ist:
yhaO-1: 5' - GCGTCGACAATGAACGCCTCCATCTCTG - 3'
yhaO-2: 5' - CAGGAACGGAGAAACACACA CGAATTTGATCGCTTCTCGC - 3'
yhaO-3: 5' - GCGAGAAGCGATCAAATTCG TGTGTGTTTCTCCGTTCCTG - 3'
yhaO-4: 5' - GCGTCGAC TTACCGCTCAAACCTTCCTG - 3'

Der Primer yhaO-2 besteht aus zwei Regionen. Die eine bindet in der Sequenz des yhaO-Gens an die Nukleotide 768 bis 787 (SEQ ID No. 1), und die andere, die 5'- terminale Region des Primers, ist reverse-komplementär zur 3'-terminalen Region des Primers yhaO-3 und bindet an die Nukleotide 21-40 dieses Primers, welcher in der Sequenz des yhaO-Gens an die Nukleotide 1988 bis 2007 (SEQ ID No. 1) bindet. Die 5'- terminale Region des Primers yhaO-3 ist wiederum reverse-komplementär zur 3'- terminalen Region des Primers yhaO-2. Zwischen beiden Primer-Bindestellen im yhaO-Gen werden somit 1200 Basenpaare deletiert.

Die für die PCR eingesetzte chromosomale E. coli K12 MG1655 DNA wird nach Herstellerangaben mit "Qiagen Genomic-tips 100/G" (QIAGEN, Hilden, Deutschland) isoliert. Ein ca. 724 bp großes DNA-Fragment aus dem 3'-Bereich der yhaO-Genregion (inklusive stromabwärts liegender Sequenzen, mit yhaO2 bezeichnet) und ein ca. 759 bp großes DNA-Fragment aus dem 5'-Bereich der yhaO-Genregion (inklusive stromaufwärts liegender Sequenzen, mit yhaO1 bezeichnet) kann mit den spezifischen Primern unter Standard-PCR-Bedingungen (Innis et al. (1990) PCR Protocols. A Guide to Methods and Applications, Academic Press) mit der Taq-DNA-Polymerase (Gibco-BRL, Eggenstein, Deutschland) amplifiziert werden. Die beiden PCR-Produkte yhaO1 und yhaO2 werden nach einer Auftrennung im 0,8%igen Agarosegel aus dem Gel isoliert und nach herkömmlichen Methoden aufgereinigt (High Pure PCR Product Purification Kit, Roche Diagnostics GmbH, Mannheim, Deutschland) und dann zusammen als Template in eine weitere PCR unter Verwendung der Primer yhaO-1 und yhaO-4 eingesetzt. Auf diesem Wege erhält man eine yhaO-Deletionskassette von 1489 Basenpaaren, welche das yhaO-Gen mit einer 1200 Basenpaar großen Deletion sowie 722 Basenpaare aus der stromaufwärts und 692 Basenpaare aus der stromabwärts liegenden Region des yhaO-Gens umfasst.

Das beschriebene yhaO-Deletionsallel wird nach der Restriktion mit dem Enzym SalI und Auftrennung im 0,8%igen Agarosegel isoliert (High Pure PCR Product Purification Kit, Roche Diagnostics GmbH, Mannheim, Deutschland) und mit dem Plasmid pKO3 (Link et al., Journal of Bacteriology 179: 6228-6237 (1997)), das ebenfalls mit dem Enzym SalI verdaut und mit Alkalischer Phosphatase (Boehringer, Mannheim, Deutschland) behandelt worden ist, mittels Quick DNA-Ligase (New England BioLabs, Frankfurt, Deutschland) ligiert.

Der Ligationsansatz wird in DH5α (Grant et al.; Proceedings of the National Academy of Sciences USA, 87 (1990) 4645-4649) transformiert (Hanahan, In. DNA Cloning. A Practical Approach. Vol. 1, ILR-Press, Cold Spring Harbor, New York, 1989) und Plasmid tragende Zellen auf LB Agar (Sambrook et al., Molecular Cloning: A Laboratory Manual. 2nd Ed., Cold Spring Harbor, New York, 1989), der mit 20 µg/ml Chloramphenicol versetzt ist, bei 30°C selektioniert.

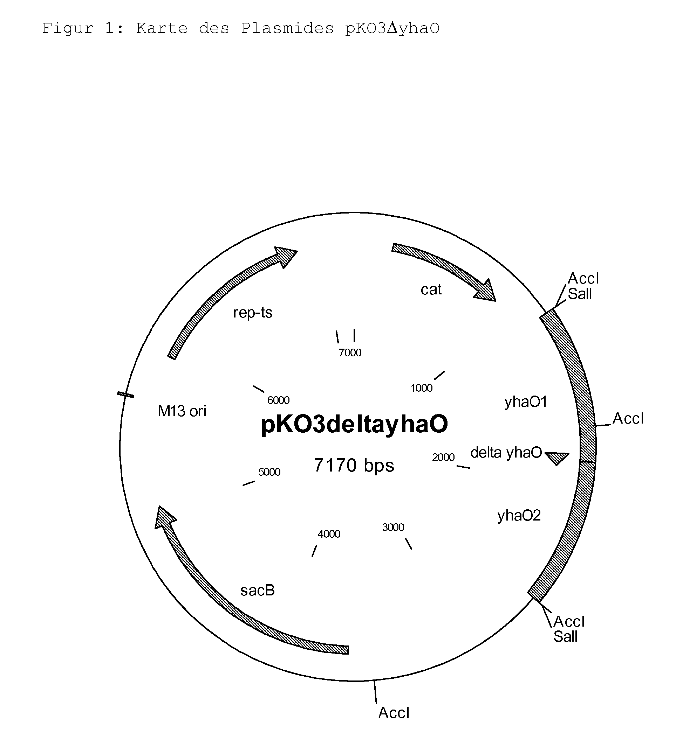

Die erfolgreiche Klonierung wird nach Plasmid DNA Isolierung und Spaltung mit dem Enzym PvuII nachgewiesen. Der entstandene Austauschvektor pKO3ΔyhaO (= pKO3deltayhaO) ist in Figur 1 dargestellt.

### Beispiel 2

### Ortsspezifische Mutagenese des yhaO-Gens in dem E. coli Stamm MG442

Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 und bei der Deutschen Sammlung für Mikroorganismen und Zellkulturen (DSMZ, Braunschweig, Deutschland) in Übereinstimmung mit dem Budapester Vertrag als DSM 16574 hinterlegt.

Für den Austausch des chromosomalen yhaO-Gens gegen das Plasmid-kodierte Deletionskonstrukt wird MG442 mit dem Plasmid pKO3ΔyhaO transformiert. Der Genaustausch erfolgt mit dem von Link et al. (Journal of Bacteriology 179: 6228-6237 (1997)) beschriebenen Selektionsverfahren und wird durch Standard-PCR-Methoden (Innis et al. (1990) PCR Protocols. A Guide to Methods and Applications, Academic Press) mit folgenden Oligonukleotid Primern verifiziert:
yhaO-1: 5' - GCGTCGACAATGAACGCCTCCATCTCTG - 3'
yhaO-4: 5' - GCGTCGAC TTACCGCTCAAACCTTCCTG - 3'

Nach erfolgtem Austausch liegt in MG442 die deletierte Form des yhaO-Allels vor. Der erhaltene Stamm wird als MG442ΔyhaO bezeichnet.

### Beispiel 3

### Herstellung von L-Threonin mit dem Stamm MG442ΔyhaO

Die Stämme MG442ΔyhaO und MG442 werden auf Minimalmedium mit der folgenden Zusammensetzung vermehrt: 3,5 g/l Na₂HPO₄*2H₂O, 1,5 g/l KH₂PO₄, 1 g/l NH₄C1, 0,1 g/l MgSO₄*7H₂O, 2 g/l Glucose, 20 g/l Agar. Die Bildung von L-Threonin wird in batch Kulturen von 10 ml, die in 100 ml Erlenmeierkolben enthalten sind, überprüft. Dazu wird 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glycerin beimpft und für 16 Stunden bei 37°C und 180 rpm auf einem ESR Inkubator der Firma Kühner AG (Birsfelden, Schweiz) inkubiert. 250 µl dieser Vorkultur werden in 10 ml Produktionsmedium (25 g/l (NH₄)₂SO₄, 2 g/l KH₂PO₄ 1 g/l MgSO₄*7H₂O, 0,03 g/l FeS0₄*7H₂O, 0,018 g/l MnSO₄*1H₂O, 30 g/l CaCO₃, 20 g/l Glycerin) überimpft und für 48 Stunden bei 37°C inkubiert. Nach der Inkubation wird die optische Dichte (OD) der Kultursuspension mit einem LP2W-Photometer der Firma Dr. Lange (Düsseldorf, Deutschland) bei einer Messwellenlänge von 660 nm bestimmt.

Anschließend wird die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

**Tabelle 1**

| Stamm | OD | L-Threonin |
|---|---|---|
| | (660 nm) | g/l |
| MG442 | 6,0 | 1,7 |
| MG442ΔyhaO | 6,1 | 2,4 |

### Beispiel 4

### Ortsspezifische Mutagenese des yhaO-Gens in dem E. coli Stamm DM1831/pMU91

Der L-Tryptophan produzierende E. coli Stamm Stamm DM1831/pMU91 ist ein durch P1-Transduktion erhältliches trpS⁺ Derivat des in der Patentschrift US-A-5,756,345 beschriebenen Escherichia coli K-12 Stammes JP6015/pMU91. pMU91 ist ein Plasmid abgeleitet von pSC101 (Cohen et al., Journal of Bacteriology 132: 734-737 (1977)), welches Tet^{R}, trpE476DCBA und serA⁺ trägt. JP6015/pMU91 ist hinterlegt als DSM 10123 bei der Deutschen Sammlung für Mikroorganismen und Zellkulturen (DSMZ, Braunschweig, Deutschland) in Übereinstimmung mit dem Budapester Vertrag.

Nach Vermehrung in Antibiotika-freiem LB-Medium für circa sechs Generationen wird ein Derivat des Stammes DM1831/pMU91 isoliert, welches das Plasmid pMU91 nicht mehr trägt. Der erhaltene Stamm ist Tetracyclin-sensitiv und wird als DM1831 bezeichnet.

Für den Austausch des chromosomalen yhaO-Gens gegen das Plasmid-kodierte Deletionskonstrukt wird DM1831 mit dem Plasmid pKO3ΔyhaO transformiert. Der Genaustausch erfolgt mit dem von Link et al. (Journal of Bacteriology 179: 6228-6237 (1997)) beschriebenen Selektionsverfahren und wird durch Standard-PCR-Methoden (Innis et al. (1990) PCR Protocols. A Guide to Methods and Applications, Academic Press) mit folgenden Oligonukleotid Primern verifiziert:
yhaO-1: 5' - GCGTCGACAATGAACGCCTCCATCTCTG - 3'
yhaO-4: 5' - GCGTCGAC TTACCGCTCAAACCTTCCTG - 3'

Nach erfolgtem Austausch liegt in DM1831 die deletierte Form des yhaO-Allels vor. Der erhaltene Stamm wird als DM1831ΔyhaO bezeichnet. Das in der Patentschrift US-A-5,756,345 beschriebene Plasmid pMU91, welches die genetischen Informationen für die Tryptophan-Produktion trägt, wird aus dem Stamm JP6015/pMU91 isoliert. Der Stamm DM1831ΔyhaO wird mit diesem Plasmid transformiert. Einer der erhaltenen Transformanten wird als DM1831ΔyhaO/pMU91 bezeichnet.

### Beispiel 5

### Herstellung von L-Tryptophan mit dem Stamm DM1831ΔyhaO/pMU91

Die Stämme DM1831ΔyhaO/pMU91 und DM1831/pMU91 werden auf LB-Medium mit der folgenden Zusammensetzung: 10 g/l Bacto-Trypton, 5 g/l Hefe-Extrakt, 10 g/l NaCl (pH-Einstellung auf 7,5 mit NaOH), 2 g/l Glucose, 20 g/l Agar und 5 mg/l Tetracyclin bei 30°C weiter vermehrt. Die Bildung von L-Tryptophan wird in batch Kulturen von 10 ml, die in 100 ml Erlenmeierkolben enthalten sind, überprüft. Dazu wird 10 ml Vorkulturmedium der folgenden Zusammensetzung: 1 g/l Hefeextrakt, 100 ml/l MOPS Puffer (10x), 10 g/l Glycerin und 5 mg/l Tetracyclin beimpft und für 16 Stunden bei 30°C und 150 rpm auf einem ESR Inkubator der Firma Kühner AG (Birsfelden, Schweiz) inkubiert.

10xMOPS Puffer wird aus den Lösungen A und B entsprechend den Tabellen 2 bis 4 hergestellt, zwei Volumen von Lösung A werden steril zu drei Volumen der Lösung B gegeben.

**Tabelle 2: Lösung A für 10xMOPS Puffer (sterilfiltriert).**

| Komponente | Konzentration |
|---|---|
| MOPS (Morpholinopropansulfonsäure) | 419 g/L |
| KOH (fest) | Zur Einstellung des pH-Wertes auf 7,4 |

**Tabelle 3: Lösung B für 10xMOPS Puffer. Sterilisiert durch Autoklavieren (30 Minuten, 121°C).**

| Komponente | Konzentration |
|---|---|
| Na₃ Citrat x 2H₂O | 2,35 g/l |
| FeSO₄ x 7H₂O | 0,22 g/l |
| NH₄Cl | 32,0 g/l |
| MgSO₄ x 7H₂O | 6,7 g/l |
| KCl | 4,0 g/l |
| CaCl₂ x 2H₂O | 0,25 mg/l |
| Stammlösung Spurenelemente (Tab. 4) | 3,33 ml/l |

**Tabelle 4: Stammlösung der Spurenelemente (in demin. Wasser) für 10xMOPS Puffer.**

| Komponente | Konzentration |
|---|---|
| (NH₄) ₆Mo₇O₂₄ x 4H₂O | 3,7 mg/l |
| H₃BO₃ | 24, mg/l |
| CoCl₂ x 6H₂O | 7,1 mg/l |
| ZnSO₄ x 7H₂O | 28,7 mg/l |
| MnCl₂ x 4H₂O | 15,8 mg/l |
| CuSO₄ x 5H₂O | 2,5 mg/l |

Je 250 µl dieser Vorkultur werden in 10 ml Produktionsmedium PM1 entsprechend Tabelle 5 überimpft und für 72 Stunden bei 30°C und 150 rpm inkubiert. Nach der Inkubation wird die optische Dichte (OD) der Kultursuspension mit einem LP2W-Photometer der Firma Dr. Lange (Düsseldorf, Deutschland) bei einer Messwellenlänge von 660 nm bestimmt.

**Tabelle 5: PM1 Medium: für Produktionstests in 100 ml Erlenmeierkolben**

| Komponente | Konzentration |
|---|---|
| 10xMOPS Puffer | 100 ml/l |
| Thiamine HCl (0,01%) | 1,0 ml/l |
| KH2PO4 (15mM) | 10 ml/l |
| Glycerin | 10 g/l |
| Tetracyclin | 5 mg/l |

Anschließend wird die Konzentration an gebildetem L-Tryptophan im steril filtrierten Kulturüberstand durch reversed phase HPLC, so wie bei Lindroth et al. (Analytical Chemistry (1979) 51: 1167-1174) beschrieben, bestimmt.

In Tabelle 6 ist das Ergebnis des Versuches dargestellt.

**Tabelle 6**

| Stamm | OD | L-Tryptophan g/l |
|---|---|---|
| | (660 nm) | |
| DM1831/pMU91 | 1,5 | 0,87 |
| DM1831ΔyhaO/pMU91 | 1,5 | 1,15 |

Kurze Beschreibung der Figuren:
- Figur 1: Karte des Plasmides pKO3ΔyhaO ( = pKO3deltayhaO)

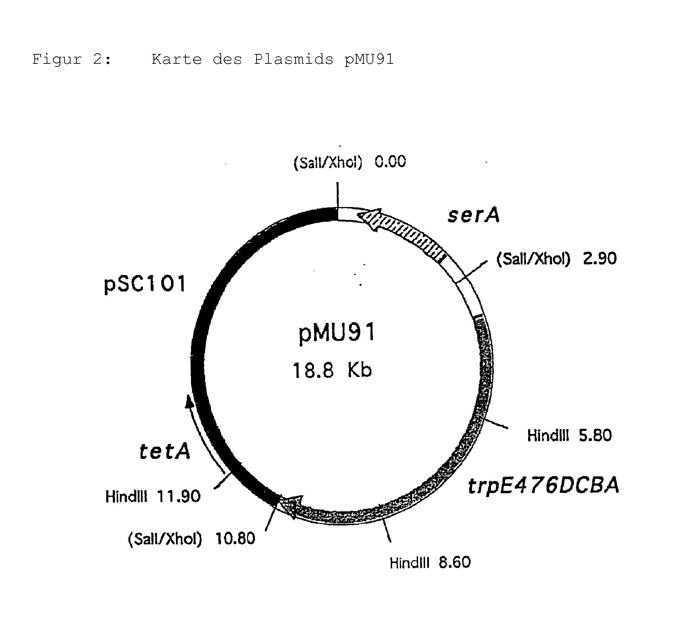

- Figur 2: Karte des Plasmides pMU91

Längenangaben sind als ca.-Angaben aufzufassen. Die verwendeten Abkürzungen und Bezeichnungen haben folgende Bedeutung:
- cat: Chloramphenicolresistenzgen
- M13 ori: Replikationsursprung
- rep-ts: temperatursensitive Replikationsregion des Plasmides pSC101
- yhaO1: Teil der 5'-Region des yhaO-Gens und der stromaufwärts liegenden Region
- delta yhaO: yhaO-Gen ohne den deletierten Bereich
- yhaO2: Teil der 3'-Region des yhaO-Gens und der stromabwärts liegenden Region
- sacB: sacB-Gen
- pSC101: Plasmidfragment pSC101
- serA : Kodierregion des serA-Gens kodierend für die D-3-Phosphoglycerat-Dehydrogenase
- trpE476DCBA :Teil des Tryptophan-Operons trpLEDCBA mit trpE476-Allel
- tetA : Tetracyclinresistenzgen

Die Abkürzungen für die Restriktionsenzyme haben folgende Bedeutung
- HindIII: Restriktionsendonuklease aus Haemophilus influenzae
- AccI: Restriktionsendonuklease aus Acinetobacter calcoaceticus
- SalI: Restriktionsendonuklease aus Streptomyces albus
XhoI: Restriktionsendonuklease aus Xanthomonas holcicola

## Patentansprüche

1. Verfahren zur Herstellung von L-Aminosäuren oder diese enthaltenden Futtermitteladditiven, **dadurch gekennzeichnet, dass** man folgende Schritte durchführt:
a) Fermentation der die gewünschte L-Aminosäure produzierenden Mikroorganismen der Familie Enterobacteriaceae, in denen mindestens der offene Leserahmen (ORF) yhaO, der für einen putativen Aminosäure/Proton-Symporter kodiert, oder die für dessen Genprodukt kodierenden Nukleotidsequenzen abgeschwächt, insbesondere ausgeschaltet wird/werden, und
b) Anreicherung der gewünschten L-Aminosäure im Medium oder in den Zellen der Mikroorganismen.

2. Verfahren gemäß Anspruch 1, **dadurch gekennzeichnet, dass** man die gewünschte L-Aminosäure isoliert, wobei gegebenenfalls weitere Bestandteile der Fermentationsbrühe und/oder die Biomasse in ihrer Gesamtheit oder Anteilen (> 0 bis 100 %) davon im isolierten Produkt verbleiben.

3. Verfahren gemäß den Ansprüchen 1 oder 2, **dadurch gekennzeichnet, dass** man L-Threonin herstellt.

4. Verfahren gemäß den Ansprüchen 1, 2, oder 3, **dadurch gekennzeichnet, dass** man Mikroorganismen einsetzt, in denen die Aktivität oder Konzentration des yhaO Genproduktes auf 0 bis 75% der Aktivität oder Konzentration des Wildtyp-Proteins oder im Elternstamm senkt.

5. Verfahren gemäß den Ansprüchen 1 bis 4, **dadurch gekennzeichnet, dass** man Mikroorganismen einsetzt, in denen man zusätzlich weitere Gene des Biosyntheseweges der gewünschten L-Aminosäure verstärkt.

6. Verfahren gemäß den Ansprüchen 1 bis 5, **dadurch gekennzeichnet, dass** man Mikroorganismen einsetzt, in denen die Stoffwechselwege zumindest teilweise ausgeschaltet sind, die die Bildung der gewünschten L-Aminosäure verringern.

7. Verfahren gemäß den Ansprüchen 1, 2, oder 3, **dadurch gekennzeichnet, dass** man die Expression des Polynukleotids, das für das Produkt des yhaO-ORFs kodiert, abschwächt, insbesondere ausschaltet.

8. Verfahren gemäß den Ansprüchen 1, 2 oder 3, **dadurch gekennzeichnet, dass** man die regulatorischen und/oder katalytischen Eigenschaften des Polypeptids (Enzymproteins) verringert, für das das Polynukleotid des yhaO-ORFs kodiert.

9. Verfahren gemäß den Ansprüchen 1 bis 8, **dadurch gekennzeichnet, dass** man zur Herstellung der gewünschten L-Aminosäuren Mikroorganismen der Familie Enterobacteriaceae fermentiert, in denen man zusätzlich gleichzeitig eines oder mehrere der Gene, ausgewählt aus der Gruppe:
9.1 mindestens ein Gen des für die Aspartatkinase, die Homoserin-Dehydrogenase, die Homoserinkinase und die Threoninsynthase kodierenden thrABC-Operons,
9.2 das für die Pyruvat-Carboxylase kodierende pyc-Gen von Corynebacterium glutamicum,
9.3 das für die Phosphoenolpyruvat-Synthase kodierende pps-Gen,
9.4 das für die Phosphoenolpyruvat-Carboxylase kodierende ppc-Gen,
9.5 die für die Untereinheiten der Pryridin-Transhydrogenase kodierenden Gene pntA und pntB,
9.6 das für das Threoninresistenz vermittelnde Protein kodierende Gen rhtC,
9.7 das für das Threonin-Export-Carrier-Protein kodierende thrE-Gen aus Corynebacterium glutamicum,
9.8 das für die Glutamat-Dehydrogenase kodierende gdhA-Gen,
9.9 das für die Phosphohistidin-Protein-Hexose-Phosphotransferase kodierende ptsH-Gen,
9.10 das für das Enzym I des Phosphotransferase-Systems kodierende ptsI-Gen,
9.11 das für die Glucose-spezifische IIA Komponente kodierende crr-Gen,
9.12 das für die Glucose-spezifische IIBC Komponente kodierende ptsG-Gen,
9.13 das für die Cystein-Synthase A kodierende cysK-Gen,
9.14 das für den Regulator des cys-Regulons kodierende cysB-Gen,
9.15 das für das Flavoprotein der NADPH-Sulfit-Reduktase kodierende cysJ-Gen,
9.16 das für das Hämoprotein der NADPH-Sulfit-Reduktase kodierende cysI-Gen,
9.17 das für die Adenylylsulfat-Reduktase kodierende cysH-Gen,
9.18 das für die Decarboxylase Untereinheit der 2-Ketoglutarat Dehydrogenase kodierende sucA-Gen,
9.19 das für die Dihydrolipoyltranssuccinase E2 Untereinheit der 2-Ketoglutarat Dehydrogenase kodierende sucB-Gen,
9.20 das für die β-Untereinheit der Succinyl-CoA Synthetase kodierende sucC-Gen,
9.21 das für die α-Untereinheit der Succinyl-CoA Synthetase kodierende sucD-Gen,
9.22 das Genprodukt des offenen Leserahmens (ORF) yibD von Escherichia coli,
9.23 das Genprodukt des offenen Leserahmens (ORF) yjcG von Escherichia coli,
9.24 das Genprodukt des offenen Leserahmens (ORF) ytfR von Escherichia coli,
9.25 das Genprodukt des offenen Leserahmens (ORF) ytfT von Escherichia coli,
9.26 das Genprodukt des offenen Leserahmens (ORF) yjfF von Escherichia coli, und
9.27 das Genprodukt des offenen Leserahmens (ORF) ytfQ von Escherichia coli,
verstärkt, insbesondere überexprimiert.

10. Verfahren gemäss den Ansprüchen 1 bis 9, **dadurch gekennzeichnet, dass** man zur Herstellung von L-Threonin Mikroorganismen der Familie Enterobacteriaceae fermentiert, in denen man zusätzlich gleichzeitig eines oder mehrere der Gene, ausgewählt aus der Gruppe:
10.1 das für die Threonin-Dehydrogenase kodierende tdh-Gen,
10.2 das für die Malat-Dehydrogenase kodierende mdh-Gen,
10.3 das Genprodukt des offenen Leserahmens (ORF) yjfA von Escherichia coli,
10.4 das Genprodukt des offenen Leserahmens (ORF) ytfP von Escherichia coli,
10.5 das für die Phosphoenolpyruvat-Carboxykinase kodierende pckA-Gen,
10.6 das für die Pyruvat-Oxidase kodierende poxB-Gen,
10.7 das für den DgsA-Regulator des Phosphotransferase-Systems kodierende dgsA-Gen,
10.8 das für den Fructose-Repressor kodierende fruR-Gen,
10.9 das für den Sigma³⁸-Faktor kodierende rpoS-Gen,
10.10 das für die Aspartat Ammonium-Lyase kodierende aspA-Gen,
10.11 das für das periplasmatische Bindeprotein eines hochaffinen ABC-Transporters für Leucin, Isoleucin und Valin kodierende livJ-Gen,
10.12 das für das periplasmatische Bindeprotein eines hochaffinen ABC-Transporters für Leucin, Isoleucin und Valin kodierende livK-Gen,
10.13 das für einen Na⁺-gekoppelten Serin/Threonin-Symporter der DAACS Familie kodierende sstT-Gen,
10.14 das für einen Threonin/Proton-Symporter der STP Familie kodierende tdcC-Gen,
10.15 das Genprodukt des offenen Leserahmens (ORF) yhjV von Escherichia coli, und
10.16 das Genprodukt des offenen Leserahmens (ORF) yqeG von Escherichia coli,
abschwächt, insbesondere ausschaltet oder die Expression verringert.

11. Verfahren gemäss den Ansprüchen 1 bis 10, **dadurch gekennzeichnet, dass** man L-Aminosäuren, ausgewählt aus der Gruppe L-Asparagin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Arginin, L-Homoserin, L-Tryptophan und L-Threonin herstellt.

12. Verfahren gemäss den Ansprüchen 1 bis 10, **dadurch gekennzeichnet, dass** man L-Aminosäuren, ausgewählt aus der Gruppe L-Isoleucin, L-Valin, L-Methionin, L-Homoserin, L-Lysin, L-Tryptophan und L-Threonin herstellt.

13. Rekombinante, bereits L-Aminosäure produzierende Mikroorganismen, in denen mindestens der offene Leserahmen (ORF) yhaO, der für einen putativen Aminosäure/Proton-Symporter kodiert, oder die für dessen Genprodukt kodierenden Nukleotidsequenzen abgeschwächt, insbesondere ausgeschaltet ist/sind.

14. Mikroorganismus gemäss Anspruch 13, **dadurch gekennzeichnet, dass** sie durch Transformation, Transduktion, Konjugation oder eine Kombination dieser Methoden hergestellt werden, mit einem Vektor, der den yhaO-ORF, ein Allel dieses ORFs oder Teile davon und/oder einen deren Expression kontrollierenden abgeschwächten Promotor enthält.

15. Mikroorganismen gemäss den Ansprüchen 13 und 14, **dadurch gekennzeichnet, dass** man zur Erzielung der Abschwächung
a) die Promotor- und Regulationsregion oder die Ribosomenbindungsstelle stromaufwärts des yhaO-ORFs mutiert, oder
b) Expresssionskassetten oder Promotoren stromaufwärts des yhaO-ORFs einbaut.

16. Mikroorganismen gemäss den Ansprüchen 13 bis 15, **dadurch gekennzeichnet, dass** die Expression des yhaO-ORFs unter der Kontrolle eines die Expression des ORFs abschwächenden Promotors steht.

17. Mikroorganismen gemäss den Ansprüchen 13 bis 16, **dadurch gekennzeichnet, dass** durch die Abschwächung des yhaO-ORFs die Konzentration oder Aktivität des yhaO-Genproduktes (Proteins) auf mindestens 0% - 75% verringert sind, bezogen auf die Aktivität oder Konzentration des Genproduktes im für den yhaO-ORF nicht rekombinanten Mikroorganismus oder Elternstamm.

18. Mikroorganismen gemäss den Ansprüchen 13 bis 17, **dadurch gekennzeichnet, dass** die Mikroorganismen aus den Gattungen Escherichia, Erwinia, Providencia und Serratia ausgewählt sind.

19. Mikroorganismen gemäss den Ansprüchen 13 bis 18, **dadurch gekennzeichnet, dass** sie eine der L-Aminosäuren, ausgewählt aus der Gruppe L-Isoleucin, L-Valin, L-Methionin, L-Homoserin, L-Lysin, L-Tryptophan und L-Threonin produzieren.

20. Mikroorganismen gemäss den Ansprüchen 13 bis 18, **dadurch gekennzeichnet, dass** sie L-Threonin produzieren.


Sequenzprotokoll

<110>  Evonik Degussa GmbH

<120>  Verfahren zur Herstellung von L-Aminosäuren unter Verwendung von
       verbesserten Stämmen der Familie Enterobacteriaceae

<130>  200900217

<160>  18

<170>  PatentIn version 3.3

<210>  1
<211>  2769
<212>  DNA
<213>  Escherichia coli

<220>
<221>  CDS
<222>  (751)..(2019)
<223>  yhaO Kodierregion

<400>  1
ccgcgggggc tattggcgcg ctgtataaaa tgaacgcctc catctctggc gcggaagtcg      60

gctgtcaggg ggagattggc gtggcctgtt caatggcggc ggcagggtta actgaactac     120

tgggcggcag tccggcgcag gtatgcaatg cggcggaaat cgcgatggag cataaccttg     180

ggctgacctg cgatccggtt gccggacagg tacaaatccc gtgcattgaa cgtaatgcca     240

ttaatgccgt gaaagcagta aacgccgcgc ggatggcgat gcgccgcacc tcggcaccgc     300

gtgtttcact cgataaagtg atcgagacga tgtatgaaac cggcaaagat atgaacgata     360

aataccgcga aacatcacgc ggaggactgg ccattaaagt ggtctgcggc tgacattgct     420

gtcagctttc atccttgggt gcacaaatgt gcaccttttt tttgtgatct gccgcaaata     480

gagaatgaat attctcctgc gattaattta gtagaaaatt cttatccatc tccgttattt     540

caaagtcttg attgtgaaga ttttctctga cctcgcgcgt atcatttctt tattctaata     600

actcgcctgc aaaaacgtag accgcatatc gccttgatgc ggctcggaaa aaaataataa     660

gccctacaaa atattgagcg agaaattatg gaaattgcat cgaataaagg cgtcattgca     720

gacgcttcga ccccggcggg tcgtgctgga atg agt gag agc gag tgg cga gaa      774
                                 Met Ser Glu Ser Glu Trp Arg Glu
                                 1               5

gcg atc aaa ttc gac agt act gac acc ggc tgg gtg att atg agt atc      822
Ala Ile Lys Phe Asp Ser Thr Asp Thr Gly Trp Val Ile Met Ser Ile
    10                  15                  20

ggg atg gcg att ggc gcg ggg att gtt ttt ctc ccg gtg cag gtc ggt      870
Gly Met Ala Ile Gly Ala Gly Ile Val Phe Leu Pro Val Gln Val Gly
25                  30                  35                  40

ttg atg gga ttg tgg gta ttt ttg ctc tca tcg gtg att ggt tac ccg      918
Leu Met Gly Leu Trp Val Phe Leu Leu Ser Ser Val Ile Gly Tyr Pro
                45                  50                  55

gca atg tat ctg ttt cag cga ttg ttt att aat acg ctg gca gaa tca      966
Ala Met Tyr Leu Phe Gln Arg Leu Phe Ile Asn Thr Leu Ala Glu Ser
            60                  65                  70

cca gaa tgt aaa gat tac ccg agc gtc att agc ggt tat tta ggt aaa      1014
Pro Glu Cys Lys Asp Tyr Pro Ser Val Ile Ser Gly Tyr Leu Gly Lys
        75                  80                  85

aac tgg ggc atc ctg tta ggt gcg ctc tat ttc gta atg ctg gtg att      1062
Asn Trp Gly Ile Leu Leu Gly Ala Leu Tyr Phe Val Met Leu Val Ile
    90                  95                  100

tgg atg ttc gtc tat tcc acc gcc atc acc aac gat agt gct tcc tac      1110
Trp Met Phe Val Tyr Ser Thr Ala Ile Thr Asn Asp Ser Ala Ser Tyr
105                 110                 115                 120

ctg cat acc ttc ggc gtg acg gaa ggg ttg ctg tca gac agt ccc ttt      1158
Leu His Thr Phe Gly Val Thr Glu Gly Leu Leu Ser Asp Ser Pro Phe
                125                 130                 135

tat ggt ctg gta ctg att tgc att ctg gtg gcg atc tcc tca cgc ggc      1206
Tyr Gly Leu Val Leu Ile Cys Ile Leu Val Ala Ile Ser Ser Arg Gly
            140                 145                 150

gag aaa ttg tta ttc aaa att tcg acc ggc atg gtg ctg acc aag ctg      1254
Glu Lys Leu Leu Phe Lys Ile Ser Thr Gly Met Val Leu Thr Lys Leu
        155                 160                 165

ctg gtg gtc gcg gcg ctg ggc gtg tcg atg gtg gga atg tgg cat ctg      1302
Leu Val Val Ala Ala Leu Gly Val Ser Met Val Gly Met Trp His Leu
    170                 175                 180

tat aac gtc ggt tcg cta ccg ccg ctg ggg ctg ctg gtg aaa aac gcc      1350
Tyr Asn Val Gly Ser Leu Pro Pro Leu Gly Leu Leu Val Lys Asn Ala
185                 190                 195                 200

att att acg ctg ccg ttt acc ctg acg tcg att ctg ttt atc cag acg      1398
Ile Ile Thr Leu Pro Phe Thr Leu Thr Ser Ile Leu Phe Ile Gln Thr
                205                 210                 215

tta agt ccg atg gtg atc tct tat cgc tcg cgg gaa aaa tca att gaa      1446
Leu Ser Pro Met Val Ile Ser Tyr Arg Ser Arg Glu Lys Ser Ile Glu
            220                 225                 230

gtg gcg cgg cat aaa gca ttg cgg gca atg aat atc gcg ttt ggc att      1494
Val Ala Arg His Lys Ala Leu Arg Ala Met Asn Ile Ala Phe Gly Ile
        235                 240                 245

ttg ttt gtc acc gtc ttt ttc tac gcc gtg tcg ttc acg ctg gcg atg      1542
Leu Phe Val Thr Val Phe Phe Tyr Ala Val Ser Phe Thr Leu Ala Met
    250                 255                 260

gga cat gac gaa gcg gta aaa gcc tat gag cag aat att tcc gcg ctg      1590
Gly His Asp Glu Ala Val Lys Ala Tyr Glu Gln Asn Ile Ser Ala Leu
265                 270                 275                 280

gcg att gcc gca cag ttt att agc ggt gac ggc gca gcg tgg gtg aaa      1638
Ala Ile Ala Ala Gln Phe Ile Ser Gly Asp Gly Ala Ala Trp Val Lys
                285                 290                 295

gtg gtc agc gtc att ctc aat atc ttt gct gtc atg act gca ttc ttt      1686
Val Val Ser Val Ile Leu Asn Ile Phe Ala Val Met Thr Ala Phe Phe
            300                 305                 310

ggc gtc tat tta ggc ttt cgc gaa gca acg caa ggg atc gta atg aac      1734
Gly Val Tyr Leu Gly Phe Arg Glu Ala Thr Gln Gly Ile Val Met Asn
        315                 320                 325

atc ctg cgt cgc aag atg cct gcc gag aag att aac gaa aat ctc gtt      1782
Ile Leu Arg Arg Lys Met Pro Ala Glu Lys Ile Asn Glu Asn Leu Val
    330                 335                 340

cag cgc ggc atc atg att ttc gcc att ttg ctg gcc tgg agc gcc atc      1830
Gln Arg Gly Ile Met Ile Phe Ala Ile Leu Leu Ala Trp Ser Ala Ile
345                 350                 355                 360

gta ctg aac gca ccg gtg ttg agc ttc acc tct atc tgt agc ccg att      1878
Val Leu Asn Ala Pro Val Leu Ser Phe Thr Ser Ile Cys Ser Pro Ile
                365                 370                 375

ttc ggc atg gta ggg tgc ctg atc ccg gcg tgg ctg gtt tac aaa gta      1926
Phe Gly Met Val Gly Cys Leu Ile Pro Ala Trp Leu Val Tyr Lys Val
            380                 385                 390

cca gca ttg cac aaa tac aaa ggg atg tct ctg tac ctg att atc gtc      1974
Pro Ala Leu His Lys Tyr Lys Gly Met Ser Leu Tyr Leu Ile Ile Val
        395                 400                 405

act ggt ttg ttg ctg tgt gtt tct ccg ttc ctg gca ttt tct tga          2019
Thr Gly Leu Leu Leu Cys Val Ser Pro Phe Leu Ala Phe Ser
    410                 415                 420

tttacctgaa attttaaggt ttttaatatg tttgattcga ctttaaatcc gttatggcag   2079

cgttacatcc tcgccgttca ggaggaagta aaaccggcgc tgggatgtac tgaaccgatt   2139

tcactggcgc tggcggcggc ggttgctgcg gcagaactgg aaggtccggt tgaacgtgta   2199

gaagcctggg tttcgccaaa tctgatgaag aacggtctgg gcgtcaccgt tcccggcacg   2259

ggaatggtgg ggctgccgat tgcggcggcg ctgggggcgt taggtggaaa tgccaacgcc   2319

gggctggaag tgctgaaaga cgcaacagcg caggcaattg ccgatgccaa agcactgctg   2379

gcggcgggga aagtctccgt taagatccag gaaccttgcg atgaaatcct cttctcacgc   2439

gccaaagtct ggaacggtga gaagtgggcg tgtgtcacca tcgtcggcgg gcataccaac   2499

attgtgcata tcgagacgca cgatggtgtg gtgtttaccc agcaggcgtg tgtggcagag   2559

ggcgagcaag agtctccgct gacggtgctt tccagaacga cgctggctga gatcctgaag   2619

ttcgtcaatg aagtcccgtt tgcggcgatc cgctttattc tcgattccgc gaagctaaat   2679

tgtgcgttat cgcaggaagg tttgagcggt aagtgggggc tgcatattgg cgcgacgctg   2739

gaaaaacagt gcgagcgcgg tttgctggcg                                     2769

<210>  2
<211>  422
<212>  PRT
<213>  Escherichia coli

<400>  2

Met Ser Glu Ser Glu Trp Arg Glu Ala Ile Lys Phe Asp Ser Thr Asp
1               5                   10                  15

Thr Gly Trp Val Ile Met Ser Ile Gly Met Ala Ile Gly Ala Gly Ile
            20                  25                  30

Val Phe Leu Pro Val Gln Val Gly Leu Met Gly Leu Trp Val Phe Leu
        35                  40                  45

Leu Ser Ser Val Ile Gly Tyr Pro Ala Met Tyr Leu Phe Gln Arg Leu
    50                  55                  60

Phe Ile Asn Thr Leu Ala Glu Ser Pro Glu Cys Lys Asp Tyr Pro Ser
65                  70                  75                  80

Val Ile Ser Gly Tyr Leu Gly Lys Asn Trp Gly Ile Leu Leu Gly Ala
                85                  90                  95

Leu Tyr Phe Val Met Leu Val Ile Trp Met Phe Val Tyr Ser Thr Ala
            100                 105                 110

Ile Thr Asn Asp Ser Ala Ser Tyr Leu His Thr Phe Gly Val Thr Glu
        115                 120                 125

Gly Leu Leu Ser Asp Ser Pro Phe Tyr Gly Leu Val Leu Ile Cys Ile
    130                 135                 140

Leu Val Ala Ile Ser Ser Arg Gly Glu Lys Leu Leu Phe Lys Ile Ser
145                 150                 155                 160

Thr Gly Met Val Leu Thr Lys Leu Leu Val Val Ala Ala Leu Gly Val
                165                 170                 175

Ser Met Val Gly Met Trp His Leu Tyr Asn Val Gly Ser Leu Pro Pro
            180                 185                 190

Leu Gly Leu Leu Val Lys Asn Ala Ile Ile Thr Leu Pro Phe Thr Leu
        195                 200                 205

Thr Ser Ile Leu Phe Ile Gln Thr Leu Ser Pro Met Val Ile Ser Tyr
    210                 215                 220

Arg Ser Arg Glu Lys Ser Ile Glu Val Ala Arg His Lys Ala Leu Arg
225                 230                 235                 240

Ala Met Asn Ile Ala Phe Gly Ile Leu Phe Val Thr Val Phe Phe Tyr
                245                 250                 255

Ala Val Ser Phe Thr Leu Ala Met Gly His Asp Glu Ala Val Lys Ala
            260                 265                 270

Tyr Glu Gln Asn Ile Ser Ala Leu Ala Ile Ala Ala Gln Phe Ile Ser
        275                 280                 285

Gly Asp Gly Ala Ala Trp Val Lys Val Val Ser Val Ile Leu Asn Ile
    290                 295                 300

Phe Ala Val Met Thr Ala Phe Phe Gly Val Tyr Leu Gly Phe Arg Glu
305                 310                 315                 320

Ala Thr Gln Gly Ile Val Met Asn Ile Leu Arg Arg Lys Met Pro Ala
                325                 330                 335

Glu Lys Ile Asn Glu Asn Leu Val Gln Arg Gly Ile Met Ile Phe Ala
            340                 345                 350

Ile Leu Leu Ala Trp Ser Ala Ile Val Leu Asn Ala Pro Val Leu Ser
        355                 360                 365

Phe Thr Ser Ile Cys Ser Pro Ile Phe Gly Met Val Gly Cys Leu Ile
    370                 375                 380

Pro Ala Trp Leu Val Tyr Lys Val Pro Ala Leu His Lys Tyr Lys Gly
385                 390                 395                 400

Met Ser Leu Tyr Leu Ile Ile Val Thr Gly Leu Leu Leu Cys Val Ser
                405                 410                 415

Pro Phe Leu Ala Phe Ser
            420

<210>  3
<211>  2841
<212>  DNA
<213>  Salmonella typhimurium

<220>
<221>  CDS
<222>  (760)..(2091)
<223>  yhaO Kodierregion

<400>  3
   aatgccaact ctatcgcccg ttatctgctg tcggcggggg cgattggtat gctgtacaag      60
   atgaacgcct cgatatccgg cgcggaagtg gggtgtcagg gcgaggttgg cgtcgcgtgt     120
   tcgatggcgg cggcggggct gacagaactg ctgggcggca gcccggcgca ggtctgtatt     180
   gcggcggaaa ttgcgatgga gcataacctt ggcctcacct gtgacccggt ggcgggtcag     240
   gtacaaatcc cttgtattga acgtaatgcg attaacgccg ttaaagcggt aaatgcggcg     300
   cgaatggcgc tacgccgtac ctctgaaccg cgggtttcgc tcgataaagt aatcgaaacc     360
   atgtacgaaa cgggtaaaga catgaacgac aaatatcgcg agacgtcgcg cggcggtctg     420
   gcgattaaag tcgtttgtgg atagacccga caggcgcaga gtgagcaggc tatgggataa     480
   cgtagaagac agaagttgag aaaagggcgc accaaagtgc gccctttttt gttgtgatac     540
   gcgtcaaata gagaaaataa caacattaaa ccaatagtaa atagaaaaga attcttaacc     600
   cacaagaaat gcaggcgttt tattggtaag tttttctctt atcgtgcgcg tatgattttc     660
   ttattacaag aaagtacctg cacctcacgt tctgcatatc gccttgctgc ggcacgaaaa     720
   ataaaaaaaa cctctaccta cacattgagc gagaatttt atg gaa tcg gca agt        774
                                              Met Glu Ser Ala Ser
                                              1               5
   aat acc agc gta atc ctc gac gca tca gcc ccg gcg cgt cgg gca ggg       822
   Asn Thr Ser Val Ile Leu Asp Ala Ser Ala Pro Ala Arg Arg Ala Gly
                   10                  15                  20
   atg act gaa agc gaa tgg cgt gaa gcc atc aaa ttt gat agt acc gac       870
   Met Thr Glu Ser Glu Trp Arg Glu Ala Ile Lys Phe Asp Ser Thr Asp
               25                  30                  35
   acc ggt tgg gtc att atg agt atc ggg atg gcg att ggc gca ggg atc      918
   Thr Gly Trp Val Ile Met Ser Ile Gly Met Ala Ile Gly Ala Gly Ile
           40                  45                  50
   gtt ttt ctt cct gtc cag gtt ggc ctg atg gga ctt tgg gtc ttt tta      966
   Val Phe Leu Pro Val Gln Val Gly Leu Met Gly Leu Trp Val Phe Leu
       55                  60                  65
   ctc tct tct att att ggc tat ccg gcc atg tac ctg ttc cag cgg ctg     1014
   Leu Ser Ser Ile Ile Gly Tyr Pro Ala Met Tyr Leu Phe Gln Arg Leu
   70                  75                  80                  85
   ttt att aat acg ctg gcg gaa tcc ccg gaa tgt aaa gac tac ccc agc     1062
   Phe Ile Asn Thr Leu Ala Glu Ser Pro Glu Cys Lys Asp Tyr Pro Ser
                   90                  95                  100
   gtg atc agc ggc tat ctg ggc aaa aac tgg ggt att tta tta ggc gcg     1110
   Val Ile Ser Gly Tyr Leu Gly Lys Asn Trp Gly Ile Leu Leu Gly Ala
               105                 110                 115
   ctg tac ttt gtg atg ctc gtc atc tgg atg ttc gtc tat tct acg gcg     1158
   Leu Tyr Phe Val Met Leu Val Ile Trp Met Phe Val Tyr Ser Thr Ala
           120                 125                 130
   att act aac gac agc gcc tct tat ctg cat act ttc ggc gta acg gaa     1206
   Ile Thr Asn Asp Ser Ala Ser Tyr Leu His Thr Phe Gly Val Thr Glu
       135                 140                 145
   gga ttg ctt tcc gat agt cca ttt tat ggt ctg gtg ctg atc tgc atc     1254
   Gly Leu Leu Ser Asp Ser Pro Phe Tyr Gly Leu Val Leu Ile Cys Ile
   150                 155                 160                 165
   ctg gtg gcg att tcc tca cgc ggt gaa aaa tta ctg ttc aaa atc tcc     1302
   Leu Val Ala Ile Ser Ser Arg Gly Glu Lys Leu Leu Phe Lys Ile Ser
                   170                 175                 180
   act ggc atg gtg ctg acc aag cta ctg gta gtg gcg gcg ctg ggc gtc     1350
   Thr Gly Met Val Leu Thr Lys Leu Leu Val Val Ala Ala Leu Gly Val
               185                 190                 195
   tct atg gtg ggt atg tgg cat ctg tat aac att ggc gcg ctg ccg ccg     1398
   Ser Met Val Gly Met Trp His Leu Tyr Asn Ile Gly Ala Leu Pro Pro
           200                 205                 210
   atg gcc ctg ctg att aaa aac gcc att att acc ctg cca ttt acg ctg     1446
   Met Ala Leu Leu Ile Lys Asn Ala Ile Ile Thr Leu Pro Phe Thr Leu
       215                 220                 225
   acc tcg att tta ttt atc cag acg tta agc cca atg gtg atc tct tac     1494
   Thr Ser Ile Leu Phe Ile Gln Thr Leu Ser Pro Met Val Ile Ser Tyr
   230                 235                 240                 245
   cgt tca cgc gaa aaa tct att gag gtc gca cgc cat aaa gcg ctg cgt     1542
   Arg Ser Arg Glu Lys Ser Ile Glu Val Ala Arg His Lys Ala Leu Arg
                   250                 255                 260
   gcc atg aat att gcg ttt ggc atc ctg ttc gtt acc gta ttt ttc tat     1590
   Ala Met Asn Ile Ala Phe Gly Ile Leu Phe Val Thr Val Phe Phe Tyr
               265                 270                 275
   gcg gtt tcc ttc acc ctg gcg atg ggg cat gac gaa gcg gtg aaa gcc     1638
   Ala Val Ser Phe Thr Leu Ala Met Gly His Asp Glu Ala Val Lys Ala
           280                 285                 290
   tac gag cag aat att tcc gcc ctg gcg atc gcc gcg caa ttt att agc     1686
   Tyr Glu Gln Asn Ile Ser Ala Leu Ala Ile Ala Ala Gln Phe Ile Ser
   295                 300                 305
   ggc gat ggc gct ggc tgg gtc aaa gtg gtt agc gta att ctg aac atc     1734
   Gly Asp Gly Ala Gly Trp Val Lys Val Val Ser Val Ile Leu Asn Ile
   310                 315                 320                 325
   ttt gcc gtc atg acc gcc ttc ttt ggc gtt tat tta gga ttc cgc gaa     1782
   Phe Ala Val Met Thr Ala Phe Phe Gly Val Tyr Leu Gly Phe Arg Glu
                   330                 335                 340
   gcg acg caa ggc att gtg atg aat atc ctg cgc cgt aaa atg ccc gcc     1830
   Ala Thr Gln Gly Ile Val Met Asn Ile Leu Arg Arg Lys Met Pro Ala
               345                 350                 355
   gag aag att aag gaa aat ctg gta cag cgc ggc att atg att ttc gct     1878
   Glu Lys Ile Lys Glu Asn Leu Val Gln Arg Gly Ile Met Ile Phe Ala
           360                 365                 370
   att ttg ctg gcc tgg agc gcc att gtg ctc aac gcg ccg gta ctg agc     1926
   Ile Leu Leu Ala Trp Ser Ala Ile Val Leu Asn Ala Pro Val Leu Ser
       375                 380                 385
   ttc acc tct att tgt agc cct atc ttc ggt atg gtg ggc tgt tta att     1974
   Phe Thr Ser Ile Cys Ser Pro Ile Phe Gly Met Val Gly Cys Leu Ile
   390                 395                 400                 405
   ccg gca tgg ttg gtc tac aag gtt ccc gca ttg cac aaa tat aaa ggc     2022
   Pro Ala Trp Leu Val Tyr Lys Val Pro Ala Leu His Lys Tyr Lys Gly
                   410                 415                 420
   gct tcg ttg tac ctg att att att acc ggt ctg ctg cta tgc gtt tct     2070
   Ala Ser Leu Tyr Leu Ile Ile Ile Thr Gly Leu Leu Leu Cys Val Ser
               425                 430                 435
   ccg ttc ctg gca ttc tct tga tatacctgag attaaggttg ttgttatgtt     2121
   Pro Phe Leu Ala Phe Ser
           440
   tgaatctaaa ataaatccat tgtggcaaag ttttattctg gccgtacagg aagaagtaaa     2181
   accggcgctg gggtgtactg agccaatttc actggcgctg gcggcagcgg cggcggcggc     2241
   tgaacttaac ggcacagttg aacgcattga cgcgtgggtt tcgcccaatc tgatgaaaaa     2301
   tggtatgggc gttaccgttc caggaacggg aatggtaggg ctaccgatcg ccgcggcgtt     2361
   gggcgcgttg ggcggtgatg cgaaagccgg gttggaggtg ttaaaagacg cttccgctaa     2421
   agccgttgcc gatgcaaaag cgatgctggc cgctggacat gtcgcggtga tgttgcagga     2481
   gccatgtaac gatattctgt tttcacgggc gaaagtgtat agcggcgata gctgggcatg     2541
   tgtcacgatc gtcggcgatc atacaaatat tgtgcggata gaaaccgaca agggtgtggt     2601
   atttacccag gccgataatg cgcaggaaga agaaaaaacc tcgccactgg gagtgctgtc     2661
   tcatacctcg ctggaagaga tcctggcgtt tgtgaatgcg gtgccctttg acgctatccg     2721
   ctttattctc gatgcggcca ggctaaacgg cgcgttgtcg caggagggac tgcgtggttc     2781
   ctgggggctg catatcggtt cgacgctggc caaacagtgc gatcgcggtc tgctggcgaa     2841

<210>  4
<211>  443
<212>  PRT
<213>  Salmonella typhimurium

<400>  4

Met Glu Ser Ala Ser Asn Thr Ser Val Ile Leu Asp Ala Ser Ala Pro
1               5                   10                  15

Ala Arg Arg Ala Gly Met Thr Glu Ser Glu Trp Arg Glu Ala Ile Lys
            20                  25                  30

Phe Asp Ser Thr Asp Thr Gly Trp Val Ile Met Ser Ile Gly Met Ala
        35                  40                  45

Ile Gly Ala Gly Ile Val Phe Leu Pro Val Gln Val Gly Leu Met Gly
    50                  55                  60

Leu Trp Val Phe Leu Leu Ser Ser Ile Ile Gly Tyr Pro Ala Met Tyr
65                  70                  75                  80

Leu Phe Gln Arg Leu Phe Ile Asn Thr Leu Ala Glu Ser Pro Glu Cys
                85                  90                  95

Lys Asp Tyr Pro Ser Val Ile Ser Gly Tyr Leu Gly Lys Asn Trp Gly
            100                 105                 110

Ile Leu Leu Gly Ala Leu Tyr Phe Val Met Leu Val Ile Trp Met Phe
        115                 120                 125

Val Tyr Ser Thr Ala Ile Thr Asn Asp Ser Ala Ser Tyr Leu His Thr
    130                 135                 140

Phe Gly Val Thr Glu Gly Leu Leu Ser Asp Ser Pro Phe Tyr Gly Leu
145                 150                 155                 160

Val Leu Ile Cys Ile Leu Val Ala Ile Ser Ser Arg Gly Glu Lys Leu
                165                 170                 175

Leu Phe Lys Ile Ser Thr Gly Met Val Leu Thr Lys Leu Leu Val Val
            180                 185                 190

Ala Ala Leu Gly Val Ser Met Val Gly Met Trp His Leu Tyr Asn Ile
        195                 200                 205

Gly Ala Leu Pro Pro Met Ala Leu Leu Ile Lys Asn Ala Ile Ile Thr
    210                 215                 220

Leu Pro Phe Thr Leu Thr Ser Ile Leu Phe Ile Gln Thr Leu Ser Pro
225                 230                 235                 240

Met Val Ile Ser Tyr Arg Ser Arg Glu Lys Ser Ile Glu Val Ala Arg
                245                 250                 255

His Lys Ala Leu Arg Ala Met Asn Ile Ala Phe Gly Ile Leu Phe Val
            260                 265                 270

Thr Val Phe Phe Tyr Ala Val Ser Phe Thr Leu Ala Met Gly His Asp
        275                 280                 285

Glu Ala Val Lys Ala Tyr Glu Gln Asn Ile Ser Ala Leu Ala Ile Ala
    290                 295                 300

Ala Gln Phe Ile Ser Gly Asp Gly Ala Gly Trp Val Lys Val Val Ser
305                 310                 315                 320

Val Ile Leu Asn Ile Phe Ala Val Met Thr Ala Phe Phe Gly Val Tyr
                325                 330                 335

Leu Gly Phe Arg Glu Ala Thr Gln Gly Ile Val Met Asn Ile Leu Arg
            340                 345                 350

Arg Lys Met Pro Ala Glu Lys Ile Lys Glu Asn Leu Val Gln Arg Gly
        355                 360                 365

Ile Met Ile Phe Ala Ile Leu Leu Ala Trp Ser Ala Ile Val Leu Asn
    370                 375                 380

Ala Pro Val Leu Ser Phe Thr Ser Ile Cys Ser Pro Ile Phe Gly Met
385                 390                 395                 400

Val Gly Cys Leu Ile Pro Ala Trp Leu Val Tyr Lys Val Pro Ala Leu
                405                 410                 415

His Lys Tyr Lys Gly Ala Ser Leu Tyr Leu Ile Ile Ile Thr Gly Leu
            420                 425                 430

Leu Leu Cys Val Ser Pro Phe Leu Ala Phe Ser
        435                 440

<210>  5
<211>  2832
<212>  DNA
<213>  Shigella flexneri

<220>
<221>  CDS
<222>  (751)..(2082)
<223>  yhaO Kodierregion

<400>  5
tggcttatta cgataagttc cgtcgtccgg taaacgagcg gtcaattgcc cgctattttc      60

tggcagcggg ggctattggc gcgctgtata aaatgaacgc ctccatctct ggcgcggagg     120

tcggctgtca gggggagatt ggcgtggcct gttcaatggc agcggcaggg ttaactgaac     180

tactgggcgg cagtccggcg caggtatgca atgcggcgga aatcgcgatg gagcataacc     240

ttgggctgac ctgcgatccg gttgccggac aggtacaaat cccgtgcatt gaacgtaatg     300

ccattaatgc cgtgaaagca gtaaacgccg cgcggatggc gatgcgccgc acctcggcac     360

cgcgtgtttc actcgataaa gtgatcgaga cgatgtatga aaccggcaaa gatatgaacg     420

ataaataccg cgaaacatca cgcggaggac tggccattaa agtggtctgc ggctgacatt 480

gctgtcagct ttcatccttg ggtgcacaaa tgtgcacctt ttttttgtga tctgccgcaa 540

atagagaatg aatattctct tgtgattaat ttagtagaaa attcttatcc atctccgtta 600

ttttaaagtc ttgattgtga agattttctc tgacctcgcg cgtatcattt ctttattcta 660

ataactcgcc tgcaaaaacg tagaccgcat atcgccttga tgcggctcgg aaaaaaataa 720

taagccctac aaaatattga gcgagaaatt atg gaa att gca tcg aat aaa ggc 774
Met Glu Ile Ala Ser Asn Lys Gly
1               5

gtc att gca gac gct tcg acc ccg gcg ggt cgt gct gga atg agt gag 822
Val Ile Ala Asp Ala Ser Thr Pro Ala Gly Arg Ala Gly Met Ser Glu
    10                  15                  20

agc gag tgg cga gaa gcg atc aaa ttc gac agt act gac acc ggc tgg 870
Ser Glu Trp Arg Glu Ala Ile Lys Phe Asp Ser Thr Asp Thr Gly Trp
25                  30                  35                  40

gtg att atg agt atc ggg atg gcg att ggc gcg ggg att gtt ttt ctc 918
Val Ile Met Ser Ile Gly Met Ala Ile Gly Ala Gly Ile Val Phe Leu
                45                  50                  55

ccg gtg cag gtc ggt ttg atg gga ttg tgg gta ttt ttg ctc tca tcg 966
Pro Val Gln Val Gly Leu Met Gly Leu Trp Val Phe Leu Leu Ser Ser
            60                  65                  70

gtg att ggt tac ccg gca atg tat ctg ttt cag cgg ttg ttt att aat 1014
Val Ile Gly Tyr Pro Ala Met Tyr Leu Phe Gln Arg Leu Phe Ile Asn
        75                  80                  85

acg ctg gca gaa tca cca gaa tgt aaa gat tac ccg agt gtc att agc 1062
Thr Leu Ala Glu Ser Pro Glu Cys Lys Asp Tyr Pro Ser Val Ile Ser
    90                  95                  100

ggt tat tta ggt aaa aac tgg ggc atc ctg tta ggt gcg ctc tat ttc 1110
Gly Tyr Leu Gly Lys Asn Trp Gly Ile Leu Leu Gly Ala Leu Tyr Phe
105                 110                 115                 120

gta atg ctg gtg att tgg atg ttc gtc tat tcc acc gcc atc acc aac 1158
Val Met Leu Val Ile Trp Met Phe Val Tyr Ser Thr Ala Ile Thr Asn
                125                 130                 135

gat agt gct tcc tat ctg cat acc ttc ggc gtg acg gaa ggg ttg ctg 1206
Asp Ser Ala Ser Tyr Leu His Thr Phe Gly Val Thr Glu Gly Leu Leu
            140                 145                 150

tca gac agt ccc ttt tat ggt ctg gta ctg att tgc att ctg gtg gcg 1254
Ser Asp Ser Pro Phe Tyr Gly Leu Val Leu Ile Cys Ile Leu Val Ala
        155                 160                 165

atc tct tca cgc ggc gag aaa ttg tta ttc aaa att tcg acc gac atg 1302
Ile Ser Ser Arg Gly Glu Lys Leu Leu Phe Lys Ile Ser Thr Asp Met
    170                 175                 180

gtg ctg acc aag ctg ctg gta gtc gct gcg ctg ggc gtg tcg atg gtg 1350
Val Leu Thr Lys Leu Leu Val Val Ala Ala Leu Gly Val Ser Met Val
185                 190                 195                 200

gga atg tgg cat ctg tat aac gtc ggt tcg cta ccg ccg ctg ggg ctg 1398
Gly Met Trp His Leu Tyr Asn Val Gly Ser Leu Pro Pro Leu Gly Leu
                205                 210                 215

ctg gtg aaa aac gcc att att acg ctg ccg ttt acc ctg acg tcg att 1446
Leu Val Lys Asn Ala Ile Ile Thr Leu Pro Phe Thr Leu Thr Ser Ile

            220                 225                 230

ctg ttt atc cag acg tta agc ccg atg gtg atc tct tat cgc tcg cgg 1494
Leu Phe Ile Gln Thr Leu Ser Pro Met Val Ile Ser Tyr Arg Ser Arg
        235                 240                 245

gaa aaa tca att gaa gtg gcg cgg cat aaa gca ttg cgg gca atg aat 1542
Glu Lys Ser Ile Glu Val Ala Arg His Lys Ala Leu Arg Ala Met Asn
    250                 255                 260

atc gcg ttt ggc att ttg ttt gtc acc gtc ttt ttc tac gcc gtg tcg 1590
Ile Ala Phe Gly Ile Leu Phe Val Thr Val Phe Phe Tyr Ala Val Ser
265                 270                 275                 280

ttc acg ctg gcg atg gga cat gac gaa gcg gta aaa gcc tac gag cag 1638
Phe Thr Leu Ala Met Gly His Asp Glu Ala Val Lys Ala Tyr Glu Gln
                285                 290                 295

aat att tcc gcg ctg gcg att gcc gca cag ttt att agc ggt gac ggc 1686
Asn Ile Ser Ala Leu Ala Ile Ala Ala Gln Phe Ile Ser Gly Asp Gly
            300                 305                 310

gca gcg tgg gtg aaa gtg gtc agc gtc att ctc aat atc ttt gct gtc 1734
Ala Ala Trp Val Lys Val Val Ser Val Ile Leu Asn Ile Phe Ala Val
        315                 320                 325

atg act gca ttc ttt ggc gtc tat tta ggc ttt cgc gaa gca acg caa 1782
Met Thr Ala Phe Phe Gly Val Tyr Leu Gly Phe Arg Glu Ala Thr Gln
    330                 335                 340

ggg atc gta atg aac atc ctg cgt cgc aag atg cct gcc gag aag att 1830
Gly Ile Val Met Asn Ile Leu Arg Arg Lys Met Pro Ala Glu Lys Ile
345                 350                 355                 360

aac gaa aat ctt gtt cag cgc ggc atc atg att ttc gcc att ttg ctg 1878
Asn Glu Asn Leu Val Gln Arg Gly Ile Met Ile Phe Ala Ile Leu Leu
                365                 370                 375

gcc tgg agc tcc atc gta ctg aac gca ccg gtg ttg agc ttc acc tct 1926
Ala Trp Ser Ser Ile Val Leu Asn Ala Pro Val Leu Ser Phe Thr Ser
            380                 385                 390

atc tgt agc ccg att ttc ggc atg gta ggg tgc ctg atc ccg gcg tgg 1974
Ile Cys Ser Pro Ile Phe Gly Met Val Gly Cys Leu Ile Pro Ala Trp
        395                 400                 405

ctg gtt tac aaa gta ccg gca ttg cac aaa tac aaa ggg atg tct ctg 2022
Leu Val Tyr Lys Val Pro Ala Leu His Lys Tyr Lys Gly Met Ser Leu
    410                 415                 420

tac ctg att atc gtc act ggt ttg ttg ctt tgt gtt tct ctg ttc ctg 2070
Tyr Leu Ile Ile Val Thr Gly Leu Leu Leu Cys Val Ser Leu Phe Leu
425                 430                 435                 440

gca ttt tct tga tttacctgaa attttaaggt ttttaatatg tttgattcga 2122
Ala Phe Ser

ctttaaatcc gttatggcag cgttacatcc tcgccgttca ggaggaagta aaaccggcgc 2182

tgggatgtac tgaaccgatt tcactggcgc tggcggcggc ggttgctgcg gcagaactgg 2242

aaggtccggt tgaacgtgta gaagcctggg tttcgccaaa tctgatgaag aacggtctgg 2302

gcgtcaccgt tcccggcacg ggaatggtgg ggctgccgat tgcggcggcg ctgggggcgt 2362

taggtggaaa tgccaacgcc gggctggaag tgctgaaaga cgcaactgcg caggcaattg 2422

ccgatgccaa agcactgctg gcggcgggga aagtctctgt taagatccag gaaccttgca 2482

atgaaatcct cttctcacgc gccaaagtct ggaacggtga gaagtgggcg tgtgtcacca 2542

tcgtcggcgg gcataccaac attgtgcata ttgagacgca caatggtgtg gtgtttaccc 2602

accaggcgtg tgtggcagag ggcgagcaag agtcgccgct gacggtgctt tccagaacga 2662

cgctggctga gatcctgaag ttcgtcaatg aagtcccatt tgcggcgatc cgctttattc 2722

tcgattccgc gaagctaaat tgtgcgttat cgcaggaagg tttgagcggt aagtgggggc 2782

tgcatattgg cgcgacgctg gaaaaacagt gcgagcgcgg tttgctggcg 2832

<210> 6
<211> 443
<212> PRT
<213> Shigella flexneri

<400> 6

Met Glu Ile Ala Ser Asn Lys Gly Val Ile Ala Asp Ala Ser Thr Pro
1               5                   10                  15

Ala Gly Arg Ala Gly Met Ser Glu Ser Glu Trp Arg Glu Ala Ile Lys
            20                  25                  30

Phe Asp Ser Thr Asp Thr Gly Trp Val Ile Met Ser Ile Gly Met Ala
        35                  40                  45

Ile Gly Ala Gly Ile Val Phe Leu Pro Val Gln Val Gly Leu Met Gly
    50                  55                  60

Leu Trp Val Phe Leu Leu Ser Ser Val Ile Gly Tyr Pro Ala Met Tyr
65                  70                  75                  80

Leu Phe Gln Arg Leu Phe Ile Asn Thr Leu Ala Glu Ser Pro Glu Cys
                85                  90                  95

Lys Asp Tyr Pro Ser Val Ile Ser Gly Tyr Leu Gly Lys Asn Trp Gly
            100                 105                 110

Ile Leu Leu Gly Ala Leu Tyr Phe Val Met Leu Val Ile Trp Met Phe
        115                 120                 125

Val Tyr Ser Thr Ala Ile Thr Asn Asp Ser Ala Ser Tyr Leu His Thr
    130                 135                 140

Phe Gly Val Thr Glu Gly Leu Leu Ser Asp Ser Pro Phe Tyr Gly Leu
145                 150                 155                 160

Val Leu Ile Cys Ile Leu Val Ala Ile Ser Ser Arg Gly Glu Lys Leu
                165                 170                 175

Leu Phe Lys Ile Ser Thr Asp Met Val Leu Thr Lys Leu Leu Val Val
            180                 185                 190

Ala Ala Leu Gly Val Ser Met Val Gly Met Trp His Leu Tyr Asn Val
        195                 200                 205

Gly Ser Leu Pro Pro Leu Gly Leu Leu Val Lys Asn Ala Ile Ile Thr
    210                 215                 220

Leu Pro Phe Thr Leu Thr Ser Ile Leu Phe Ile Gln Thr Leu Ser Pro
225                 230                 235                 240

Met Val Ile Ser Tyr Arg Ser Arg Glu Lys Ser Ile Glu Val Ala Arg
                245                 250                 255

His Lys Ala Leu Arg Ala Met Asn Ile Ala Phe Gly Ile Leu Phe Val
            260                 265                 270

Thr Val Phe Phe Tyr Ala Val Ser Phe Thr Leu Ala Met Gly His Asp
        275                 280                 285

Glu Ala Val Lys Ala Tyr Glu Gln Asn Ile Ser Ala Leu Ala Ile Ala
    290                 295                 300

Ala Gln Phe Ile Ser Gly Asp Gly Ala Ala Trp Val Lys Val Val Ser
305                 310                 315                 320

Val Ile Leu Asn Ile Phe Ala Val Met Thr Ala Phe Phe Gly Val Tyr
                325                 330                 335

Leu Gly Phe Arg Glu Ala Thr Gln Gly Ile Val Met Asn Ile Leu Arg
            340                 345                 350

Arg Lys Met Pro Ala Glu Lys Ile Asn Glu Asn Leu Val Gln Arg Gly
        355                 360                 365

Ile Met Ile Phe Ala Ile Leu Leu Ala Trp Ser Ser Ile Val Leu Asn
    370                 375                 380

Ala Pro Val Leu Ser Phe Thr Ser Ile Cys Ser Pro Ile Phe Gly Met
385                 390                 395                 400

Val Gly Cys Leu Ile Pro Ala Trp Leu Val Tyr Lys Val Pro Ala Leu
                405                 410                 415

His Lys Tyr Lys Gly Met Ser Leu Tyr Leu Ile Ile Val Thr Gly Leu
            420                 425                 430

Leu Leu Cys Val Ser Leu Phe Leu Ala Phe Ser
        435                 440

<210>  7
<211>  2463
<212>  DNA
<213>  Escherichia coli

<220>
<221> CDS
<222> (1)..(2463)
<223> thrA-Kodierregion

<400> 7
atg cga gtg ttg aag ttc ggc ggt aca tca gtg gca aat gca gaa cgt 48
Met Arg Val Leu Lys Phe Gly Gly Thr Ser Val Ala Asn Ala Glu Arg
1               5                   10                  15

ttt ctg cgt gtt gcc gat att ctg gaa agc aat gcc agg cag ggg cag 96
Phe Leu Arg Val Ala Asp Ile Leu Glu Ser Asn Ala Arg Gln Gly Gln
            20                  25                  30

gtg gcc acc gtc ctc tct gcc ccc gcc aaa atc acc aac cac ctg gtg 144
Val Ala Thr Val Leu Ser Ala Pro Ala Lys Ile Thr Asn His Leu Val
        35                  40                  45

gcg atg att gaa aaa acc att agc ggc cag gat gct tta ccc aat atc 192
Ala Met Ile Glu Lys Thr Ile Ser Gly Gln Asp Ala Leu Pro Asn Ile
    50                  55                  60

agc gat gcc gaa cgt att ttt gcc gaa ctt ttg acg gga ctc gcc gcc 240
Ser Asp Ala Glu Arg Ile Phe Ala Glu Leu Leu Thr Gly Leu Ala Ala
65                  70                  75                  80

gcc cag ccg ggg ttc ccg ctg gcg caa ttg aaa act ttc gtc gat cag 288
Ala Gln Pro Gly Phe Pro Leu Ala Gln Leu Lys Thr Phe Val Asp Gln
                85                  90                  95

gaa ttt gcc caa ata aaa cat gtc ctg cat ggc att agt ttg ttg ggg 336
Glu Phe Ala Gln Ile Lys His Val Leu His Gly Ile Ser Leu Leu Gly
            100                 105                 110

cag tgc ccg gat agc atc aac gct gcg ctg att tgc cgt ggc gag aaa 384
Gln Cys Pro Asp Ser Ile Asn Ala Ala Leu Ile Cys Arg Gly Glu Lys
        115                 120                 125

atg tcg atc gcc att atg gcc ggc gta tta gaa gcg cgc ggt cac aac 432
Met Ser Ile Ala Ile Met Ala Gly Val Leu Glu Ala Arg Gly His Asn
    130                 135                 140

gtt act gtt atc gat ccg gtc gaa aaa ctg ctg gca gtg ggg cat tac 480
Val Thr Val Ile Asp Pro Val Glu Lys Leu Leu Ala Val Gly His Tyr
145                 150                 155                 160

ctc gaa tct acc gtc gat att gct gag tcc acc cgc cgt att gcg gca 528
Leu Glu Ser Thr Val Asp Ile Ala Glu Ser Thr Arg Arg Ile Ala Ala
                165                 170                 175

agc cgc att ccg gct gat cac atg gtg ctg atg gca ggt ttc acc gcc 576
Ser Arg Ile Pro Ala Asp His Met Val Leu Met Ala Gly Phe Thr Ala
            180                 185                 190

ggt aat gaa aaa ggc gaa ctg gtg gtg ctt gga cgc aac ggt tcc gac 624
Gly Asn Glu Lys Gly Glu Leu Val Val Leu Gly Arg Asn Gly Ser Asp
        195                 200                 205

tac tct gct gcg gtg ctg gct gcc tgt tta cgc gcc gat tgt tgc gag 672
Tyr Ser Ala Ala Val Leu Ala Ala Cys Leu Arg Ala Asp Cys Cys Glu
    210                 215                 220

att tgg acg gac gtt gac ggg gtc tat acc tgc gac ccg cgt cag gtg 720
Ile Trp Thr Asp Val Asp Gly Val Tyr Thr Cys Asp Pro Arg Gln Val
225                 230                 235                 240

ccc gat gcg agg ttg ttg aag tcg atg tcc tac cag gaa gcg atg gag 768
Pro Asp Ala Arg Leu Leu Lys Ser Met Ser Tyr Gln Glu Ala Met Glu

                245                 250                 255

ctt tcc tac ttc ggc gct aaa gtt ctt cac ccc cgc acc att acc ccc 816
Leu Ser Tyr Phe Gly Ala Lys Val Leu His Pro Arg Thr Ile Thr Pro
            260                 265                 270

atc gcc cag ttc cag atc cct tgc ctg att aaa aat acc gga aat cct 864
Ile Ala Gln Phe Gln Ile Pro Cys Leu Ile Lys Asn Thr Gly Asn Pro
        275                 280                 285

caa gca cca ggt acg ctc att ggt gcc agc cgt gat gaa gac gaa tta 912
Gln Ala Pro Gly Thr Leu Ile Gly Ala Ser Arg Asp Glu Asp Glu Leu
    290                 295                 300

ccg gtc aag ggc att tcc aat ctg aat aac atg gca atg ttc agc gtt 960
Pro Val Lys Gly Ile Ser Asn Leu Asn Asn Met Ala Met Phe Ser Val
305                 310                 315                 320

tct ggt ccg ggg atg aaa ggg atg gtc ggc atg gcg gcg cgc gtc ttt 1008
Ser Gly Pro Gly Met Lys Gly Met Val Gly Met Ala Ala Arg Val Phe
                325                 330                 335

gca gcg atg tca cgc gcc cgt att tcc gtg gtg ctg att acg caa tca 1056
Ala Ala Met Ser Arg Ala Arg Ile Ser Val Val Leu Ile Thr Gln Ser
            340                 345                 350

tct tcc gaa tac agc atc agt ttc tgc gtt cca caa agc gac tgt gtg 1104
Ser Ser Glu Tyr Ser Ile Ser Phe Cys Val Pro Gln Ser Asp Cys Val
        355                 360                 365

cga gct gaa cgg gca atg cag gaa gag ttc tac ctg gaa ctg aaa gaa 1152
Arg Ala Glu Arg Ala Met Gln Glu Glu Phe Tyr Leu Glu Leu Lys Glu
    370                 375                 380

ggc tta ctg gag ccg ctg gca gtg acg gaa cgg ctg gcc att atc tcg 1200
Gly Leu Leu Glu Pro Leu Ala Val Thr Glu Arg Leu Ala Ile Ile Ser
385                 390                 395                 400

gtg gta ggt gat ggt atg cgc acc ttg cgt ggg atc tcg gcg aaa ttc 1248
Val Val Gly Asp Gly Met Arg Thr Leu Arg Gly Ile Ser Ala Lys Phe
                405                 410                 415

ttt gcc gca ctg gcc cgc gcc aat atc aac att gtc gcc att gct cag 1296
Phe Ala Ala Leu Ala Arg Ala Asn Ile Asn Ile Val Ala Ile Ala Gln
            420                 425                 430

gga tct tct gaa cgc tca atc tct gtc gtg gta aat aac gat gat gcg 1344
Gly Ser Ser Glu Arg Ser Ile Ser Val Val Val Asn Asn Asp Asp Ala
        435                 440                 445

acc act ggc gtg cgc gtt act cat cag atg ctg ttc aat acc gat cag 1392
Thr Thr Gly Val Arg Val Thr His Gln Met Leu Phe Asn Thr Asp Gln
    450                 455                 460

gtt atc gaa gtg ttt gtg att ggc gtc ggt ggc gtt ggc ggt gcg ctg 1440
Val Ile Glu Val Phe Val Ile Gly Val Gly Gly Val Gly Gly Ala Leu
465                 470                 475                 480

ctg gag caa ctg aag cgt cag caa agc tgg ctg aag aat aaa cat atc 1488
Leu Glu Gln Leu Lys Arg Gln Gln Ser Trp Leu Lys Asn Lys His Ile
                485                 490                 495

gac tta cgt gtc tgc ggt gtt gcc aac tcg aag gct ctg ctc acc aat 1536
Asp Leu Arg Val Cys Gly Val Ala Asn Ser Lys Ala Leu Leu Thr Asn
            500                 505                 510

gta cat ggc ctt aat ctg gaa aac tgg cag gaa gaa ctg gcg caa gcc 1584
Val His Gly Leu Asn Leu Glu Asn Trp Gln Glu Glu Leu Ala Gln Ala
        515                 520                 525

aaa gag ccg ttt aat ctc ggg cgc tta att cgc ctc gtg aaa gaa tat     1632
Lys Glu Pro Phe Asn Leu Gly Arg Leu Ile Arg Leu Val Lys Glu Tyr
    530                 535                 540

cat ctg ctg aac ccg gtc att gtt gac tgc act tcc agc cag gca gtg     1680
His Leu Leu Asn Pro Val Ile Val Asp Cys Thr Ser Ser Gln Ala Val
545                 550                 555                 560

gcg gat caa tat gcc gac ttc ctg cgc gaa ggt ttc cac gtt gtc acg     1728
Ala Asp Gln Tyr Ala Asp Phe Leu Arg Glu Gly Phe His Val Val Thr
                565                 570                 575

ccg aac aaa aag gcc aac acc tcg tcg atg gat tac tac cat cag ttg     1776
Pro Asn Lys Lys Ala Asn Thr Ser Ser Met Asp Tyr Tyr His Gln Leu
            580                 585                 590

cgt tat gcg gcg gaa aaa tcg cgg cgt aaa ttc ctc tat gac acc aac     1824
Arg Tyr Ala Ala Glu Lys Ser Arg Arg Lys Phe Leu Tyr Asp Thr Asn
        595                 600                 605

gtt ggg gct gga tta ccg gtt att gag aac ctg caa aat ctg ctc aat     1872
Val Gly Ala Gly Leu Pro Val Ile Glu Asn Leu Gln Asn Leu Leu Asn
    610                 615                 620

gca ggt gat gaa ttg atg aag ttc tcc ggc att ctt tct ggt tcg ctt     1920
Ala Gly Asp Glu Leu Met Lys Phe Ser Gly Ile Leu Ser Gly Ser Leu
625                 630                 635                 640

tct tat atc ttc ggc aag tta gac gaa ggc atg agt ttc tcc gag gcg     1968
Ser Tyr Ile Phe Gly Lys Leu Asp Glu Gly Met Ser Phe Ser Glu Ala
                645                 650                 655

acc acg ctg gcg cgg gaa atg ggt tat acc gaa ccg gac ccg cga gat     2016
Thr Thr Leu Ala Arg Glu Met Gly Tyr Thr Glu Pro Asp Pro Arg Asp
            660                 665                 670

gat ctt tct ggt atg gat gtg gcg cgt aaa cta ttg att ctc gct cgt     2064
Asp Leu Ser Gly Met Asp Val Ala Arg Lys Leu Leu Ile Leu Ala Arg
        675                 680                 685

gaa acg gga cgt gaa ctg gag ctg gcg gat att gaa att gaa cct gtg     2112
Glu Thr Gly Arg Glu Leu Glu Leu Ala Asp Ile Glu Ile Glu Pro Val
    690                 695                 700

ctg ccc gca gag ttt aac gcc gag ggt gat gtt gcc gct ttt atg gcg     2160
Leu Pro Ala Glu Phe Asn Ala Glu Gly Asp Val Ala Ala Phe Met Ala
705                 710                 715                 720

aat ctg tca caa ctc gac gat ctc ttt gcc gcg cgc gtg gcg aag gcc     2208
Asn Leu Ser Gln Leu Asp Asp Leu Phe Ala Ala Arg Val Ala Lys Ala
                725                 730                 735

cgt gat gaa gga aaa gtt ttg cgc tat gtt ggc aat att gat gaa gat     2256
Arg Asp Glu Gly Lys Val Leu Arg Tyr Val Gly Asn Ile Asp Glu Asp
            740                 745                 750

ggc gtc tgc cgc gtg aag att gcc gaa gtg gat ggt aat gat ccg ctg     2304
Gly Val Cys Arg Val Lys Ile Ala Glu Val Asp Gly Asn Asp Pro Leu
        755                 760                 765

ttc aaa gtg aaa aat ggc gaa aac gcc ctg gcc ttc tat agc cac tat     2352
Phe Lys Val Lys Asn Gly Glu Asn Ala Leu Ala Phe Tyr Ser His Tyr
    770                 775                 780

tat cag ccg ctg ccg ttg gta ctg cgc gga tat ggt gcg ggc aat gac     2400
Tyr Gln Pro Leu Pro Leu Val Leu Arg Gly Tyr Gly Ala Gly Asn Asp
785                 790                 795                 800

gtt aca gct gcc ggt gtc ttt gct gat ctg cta cgt acc ctc tca tgg     2448
Val Thr Ala Ala Gly Val Phe Ala Asp Leu Leu Arg Thr Leu Ser Trp
                805                 810                 815

aag tta gga gtc tga                                                 2463
Lys Leu Gly Val
            820

<210>  8
<211>  820
<212>  PRT
<213>  Escherichia coli

<400>  8

Met Arg Val Leu Lys Phe Gly Gly Thr Ser Val Ala Asn Ala Glu Arg
1               5                   10                  15

Phe Leu Arg Val Ala Asp Ile Leu Glu Ser Asn Ala Arg Gln Gly Gln
            20                  25                  30

Val Ala Thr Val Leu Ser Ala Pro Ala Lys Ile Thr Asn His Leu Val
        35                  40                  45

Ala Met Ile Glu Lys Thr Ile Ser Gly Gln Asp Ala Leu Pro Asn Ile
    50                  55                  60

Ser Asp Ala Glu Arg Ile Phe Ala Glu Leu Leu Thr Gly Leu Ala Ala
65                  70                  75                  80

Ala Gln Pro Gly Phe Pro Leu Ala Gln Leu Lys Thr Phe Val Asp Gln
                85                  90                  95

Glu Phe Ala Gln Ile Lys His Val Leu His Gly Ile Ser Leu Leu Gly
            100                 105                 110

Gln Cys Pro Asp Ser Ile Asn Ala Ala Leu Ile Cys Arg Gly Glu Lys
        115                 120                 125

Met Ser Ile Ala Ile Met Ala Gly Val Leu Glu Ala Arg Gly His Asn
    130                 135                 140

Val Thr Val Ile Asp Pro Val Glu Lys Leu Leu Ala Val Gly His Tyr
145                 150                 155                 160

Leu Glu Ser Thr Val Asp Ile Ala Glu Ser Thr Arg Arg Ile Ala Ala
                165                 170                 175

Ser Arg Ile Pro Ala Asp His Met Val Leu Met Ala Gly Phe Thr Ala
            180                 185                 190

Gly Asn Glu Lys Gly Glu Leu Val Val Leu Gly Arg Asn Gly Ser Asp
        195                 200                 205

Tyr Ser Ala Ala Val Leu Ala Ala Cys Leu Arg Ala Asp Cys Cys Glu

210                 215                 220

Ile Trp Thr Asp Val Asp Gly Val Tyr Thr Cys Asp Pro Arg Gln Val
225                 230                 235                 240

Pro Asp Ala Arg Leu Leu Lys Ser Met Ser Tyr Gln Glu Ala Met Glu
                245                 250                 255

Leu Ser Tyr Phe Gly Ala Lys Val Leu His Pro Arg Thr Ile Thr Pro
            260                 265                 270

Ile Ala Gln Phe Gln Ile Pro Cys Leu Ile Lys Asn Thr Gly Asn Pro
        275                 280                 285

Gln Ala Pro Gly Thr Leu Ile Gly Ala Ser Arg Asp Glu Asp Glu Leu
    290                 295                 300

Pro Val Lys Gly Ile Ser Asn Leu Asn Asn Met Ala Met Phe Ser Val
305                 310                 315                 320

Ser Gly Pro Gly Met Lys Gly Met Val Gly Met Ala Ala Arg Val Phe
                325                 330                 335

Ala Ala Met Ser Arg Ala Arg Ile Ser Val Val Leu Ile Thr Gln Ser
            340                 345                 350

Ser Ser Glu Tyr Ser Ile Ser Phe Cys Val Pro Gln Ser Asp Cys Val
        355                 360                 365

Arg Ala Glu Arg Ala Met Gln Glu Glu Phe Tyr Leu Glu Leu Lys Glu
    370                 375                 380

Gly Leu Leu Glu Pro Leu Ala Val Thr Glu Arg Leu Ala Ile Ile Ser
385                 390                 395                 400

Val Val Gly Asp Gly Met Arg Thr Leu Arg Gly Ile Ser Ala Lys Phe
                405                 410                 415

Phe Ala Ala Leu Ala Arg Ala Asn Ile Asn Ile Val Ala Ile Ala Gln
            420                 425                 430

Gly Ser Ser Glu Arg Ser Ile Ser Val Val Val Asn Asn Asp Asp Ala
        435                 440                 445

Thr Thr Gly Val Arg Val Thr His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Leu Glu Gln Leu Lys Arg Gln Gln Ser Trp Leu Lys Asn Lys His Ile
                485                 490                 495

Asp Leu Arg Val Cys Gly Val Ala Asn Ser Lys Ala Leu Leu Thr Asn
            500                 505                 510

Val His Gly Leu Asn Leu Glu Asn Trp Gln Glu Glu Leu Ala Gln Ala
        515                 520                 525

Lys Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Ala Asp Phe Leu Arg Glu Gly Phe His Val Val Thr
                565                 570                 575

Pro Asn Lys Lys Ala Asn Thr Ser Ser Met Asp Tyr Tyr His Gln Leu
            580                 585                 590

Arg Tyr Ala Ala Glu Lys Ser Arg Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Met Lys Phe Ser Gly Ile Leu Ser Gly Ser Leu
625                 630                 635                 640

Ser Tyr Ile Phe Gly Lys Leu Asp Glu Gly Met Ser Phe Ser Glu Ala
                645                 650                 655

Thr Thr Leu Ala Arg Glu Met Gly Tyr Thr Glu Pro Asp Pro Arg Asp
            660                 665                 670

Asp Leu Ser Gly Met Asp Val Ala Arg Lys Leu Leu Ile Leu Ala Arg
        675                 680                 685

Glu Thr Gly Arg Glu Leu Glu Leu Ala Asp Ile Glu Ile Glu Pro Val
    690                 695                 700

Leu Pro Ala Glu Phe Asn Ala Glu Gly Asp Val Ala Ala Phe Met Ala
705                 710                 715                 720

Asn Leu Ser Gln Leu Asp Asp Leu Phe Ala Ala Arg Val Ala Lys Ala
                725                 730                 735

Arg Asp Glu Gly Lys Val Leu Arg Tyr Val Gly Asn Ile Asp Glu Asp
            740                 745                 750

Gly Val Cys Arg Val Lys Ile Ala Glu Val Asp Gly Asn Asp Pro Leu
        755                 760                 765

Phe Lys Val Lys Asn Gly Glu Asn Ala Leu Ala Phe Tyr Ser His Tyr
    770                 775                 780

Tyr Gln Pro Leu Pro Leu Val Leu Arg Gly Tyr Gly Ala Gly Asn Asp
785                 790                 795                 800

Val Thr Ala Ala Gly Val Phe Ala Asp Leu Leu Arg Thr Leu Ser Trp
                805                 810                 815

Lys Leu Gly Val
            820

<210>  9
<211>  2460
<212>  DNA
<213>  Serratia marcescens

<220>
<221>  CDS
<222>  (1)..(2460)
<223>  thrA-Kodierregion

<400>  9
atg cga gtg ctg aaa ttt ggc gga acc tcg gta gcg aat gcg gaa cgt       48
Met Arg Val Leu Lys Phe Gly Gly Thr Ser Val Ala Asn Ala Glu Arg
1               5                   10                  15

ttt ctg cgc gtc gcc gac atc atg gag agt aac gcg cgt cag gga cag       96
Phe Leu Arg Val Ala Asp Ile Met Glu Ser Asn Ala Arg Gln Gly Gln
            20                  25                  30

gta gcc acg gtc ttg tcc gcc ccc gca aag atc acc aac cat ctg gtg      144
Val Ala Thr Val Leu Ser Ala Pro Ala Lys Ile Thr Asn His Leu Val
        35                  40                  45

gcg atg atc gac aaa acg gtg gcg ggc cag gac att ctg ccg aat atg      192
Ala Met Ile Asp Lys Thr Val Ala Gly Gln Asp Ile Leu Pro Asn Met
    50                  55                  60

agc gac gcc gag cgg atc ttt gcc gac ctg ctg agc gga ctg gcg cag      240
Ser Asp Ala Glu Arg Ile Phe Ala Asp Leu Leu Ser Gly Leu Ala Gln
65                  70                  75                  80

gcg ctg ccg ggc ttt gaa tac gat cgt ttg aaa ggc gtg gtc gat cag      288
Ala Leu Pro Gly Phe Glu Tyr Asp Arg Leu Lys Gly Val Val Asp Gln
                85                  90                  95

gaa ttc gcg cag ctc aaa cag gtg ctg cat ggc gtc tcg ctg ctg ggg      336
Glu Phe Ala Gln Leu Lys Gln Val Leu His Gly Val Ser Leu Leu Gly
            100                 105                 110

cag tgc ccg gac agc gtg aac gcg gcg atc atc tgc cgt ggc gaa aag      384
Gln Cys Pro Asp Ser Val Asn Ala Ala Ile Ile Cys Arg Gly Glu Lys
        115                 120                 125

ctc tcc atc gcc atc atg gaa ggg gtg ttc cgc gcc aag ggt tat ccg      432
Leu Ser Ile Ala Ile Met Glu Gly Val Phe Arg Ala Lys Gly Tyr Pro
    130                 135                 140

gtc acg gtg atc aac ccg gtg gag aaa ctg ctg gcg cag ggc cac tac      480
Val Thr Val Ile Asn Pro Val Glu Lys Leu Leu Ala Gln Gly His Tyr
145                 150                 155                 160

ctg gag tcc acc gtg gac atc gcc gag tct acg ctg cgc atc gcc gcc      528
Leu Glu Ser Thr Val Asp Ile Ala Glu Ser Thr Leu Arg Ile Ala Ala
                165                 170                 175

gcg gcg atc ccg gcc gat cac atc gtg ctg atg gcc ggt ttc acc gcc      576
Ala Ala Ile Pro Ala Asp His Ile Val Leu Met Ala Gly Phe Thr Ala
            180                 185                 190

ggt aat gat aag ggc gag ctg gtg gtg ctg ggc cgc aac ggc tcc gac      624
Gly Asn Asp Lys Gly Glu Leu Val Val Leu Gly Arg Asn Gly Ser Asp
        195                 200                 205

tat tcc gcc gcg gtg ctg gcc gcc tgc ctg cgc gcc gac tgt tgc gag      672
Tyr Ser Ala Ala Val Leu Ala Ala Cys Leu Arg Ala Asp Cys Cys Glu
    210                 215                 220

atc tgg acc gac gtc gac ggc gtt tat acc tgc gat ccg cgc acc gtg      720
Ile Trp Thr Asp Val Asp Gly Val Tyr Thr Cys Asp Pro Arg Thr Val
225                 230                 235                 240

ccg gac gcc agg tta ctg aaa tcg atg tcg tac cag gaa gcg atg gag      768
Pro Asp Ala Arg Leu Leu Lys Ser Met Ser Tyr Gln Glu Ala Met Glu
                245                 250                 255

ctt tcc tat ttc ggc gcc aaa gtg tta cac ccc cgc acc atc act ccg      816
Leu Ser Tyr Phe Gly Ala Lys Val Leu His Pro Arg Thr Ile Thr Pro
            260                 265                 270

att gcc cag ttt caa atc cct tgc ctg att aaa aac acc tcc aac ccg      864
Ile Ala Gln Phe Gln Ile Pro Cys Leu Ile Lys Asn Thr Ser Asn Pro
        275                 280                 285

cag gcc ccc ggc acg ctg atc ggc aaa gac agc acc gat gcc gat atg      912
Gln Ala Pro Gly Thr Leu Ile Gly Lys Asp Ser Thr Asp Ala Asp Met
    290                 295                 300

ccg gtg aag ggc atc acc aac ctg aac aac atg gcg atg atc aac gtc      960
Pro Val Lys Gly Ile Thr Asn Leu Asn Asn Met Ala Met Ile Asn Val
305                 310                 315                 320

tcc ggc ccg ggc atg aaa ggc atg gtc ggc atg gcc gca cgg gtc ttc     1008
Ser Gly Pro Gly Met Lys Gly Met Val Gly Met Ala Ala Arg Val Phe
                325                 330                 335

gcc gtg atg tcg cgc gcc ggc att tcg gtg gtg ctg atc acc caa tcc     1056
Ala Val Met Ser Arg Ala Gly Ile Ser Val Val Leu Ile Thr Gln Ser
            340                 345                 350

tct tcc gaa tac agc atc agc ttc tgc gtg ccg cag ggt gaa ctg cag     1104
Ser Ser Glu Tyr Ser Ile Ser Phe Cys Val Pro Gln Gly Glu Leu Gln
        355                 360                 365

cgc gcc cgt cgc gcg ctg gaa gag gag ttc tat ctg gag ctg aaa gac     1152
Arg Ala Arg Arg Ala Leu Glu Glu Glu Phe Tyr Leu Glu Leu Lys Asp
    370                 375                 380

ggc gtg ctg gat ccg ctg gac gtg atg gaa cgc ctg gcg atc atc tcg     1200
Gly Val Leu Asp Pro Leu Asp Val Met Glu Arg Leu Ala Ile Ile Ser
385                 390                 395                 400

gtg gtt ggc gac ggc atg cgc acc ctg cgc ggc att tcc gca cgc ttc     1248
Val Val Gly Asp Gly Met Arg Thr Leu Arg Gly Ile Ser Ala Arg Phe
                405                 410                 415

ttc tcc gcg ctg gcg cgc gcc aac atc aat atc gtt gcc atc gcc caa     1296
Phe Ser Ala Leu Ala Arg Ala Asn Ile Asn Ile Val Ala Ile Ala Gln
            420                 425                 430

ggg tct tcc gaa cgt tcc att tcg gtg gtg gtc agc aat gat tcc gcc     1344

Gly Ser Ser Glu Arg Ser Ile Ser Val Val Val Ser Asn Asp Ser Ala
        435                 440                 445

acc acc ggc gtg cgc gtc agc cac cag atg ctg ttc aac acc gat cag      1392
Thr Thr Gly Val Arg Val Ser His Gln Met Leu Phe Asn Thr Asp Gln
    450                 455                 460

gtg atc gaa gtg ttc gtc atc ggc gtc ggc ggc gtc ggc ggg gcg ctg      1440
Val Ile Glu Val Phe Val Ile Gly Val Gly Gly Val Gly Gly Ala Leu
465                 470                 475                 480

atc gag cag atc tat cgc cag cag ccg tgg ctg aag caa aaa cat atc      1488
Ile Glu Gln Ile Tyr Arg Gln Gln Pro Trp Leu Lys Gln Lys His Ile
                485                 490                 495

gat ctg cgg gtg tgc ggc atc gcc aac tcg cgc gtg atg ctg acc aat      1536
Asp Leu Arg Val Cys Gly Ile Ala Asn Ser Arg Val Met Leu Thr Asn
            500                 505                 510

gtg cac ggc atc gcg ctg gac agc tgg cgc gac gcg ctg gcc ggc gcg      1584
Val His Gly Ile Ala Leu Asp Ser Trp Arg Asp Ala Leu Ala Gly Ala
        515                 520                 525

cag gag ccg ttc aat ctc ggc cgt ctg atc cgg ctg gtg aag gaa tat      1632
Gln Glu Pro Phe Asn Leu Gly Arg Leu Ile Arg Leu Val Lys Glu Tyr
    530                 535                 540

cac ctg ctg aac ccg gtg atc gtc gac tgt acc tcc agt cag gcg gtg      1680
His Leu Leu Asn Pro Val Ile Val Asp Cys Thr Ser Ser Gln Ala Val
545                 550                 555                 560

gcc gat cag tac gtg gac ttc ctg gcg gac ggc ttc cat gtg gtg acg      1728
Ala Asp Gln Tyr Val Asp Phe Leu Ala Asp Gly Phe His Val Val Thr
                565                 570                 575

ccg aac aaa aag gcc aac acc tcg tcg atg aac tat tat cag caa ctg      1776
Pro Asn Lys Lys Ala Asn Thr Ser Ser Met Asn Tyr Tyr Gln Gln Leu
            580                 585                 590

cgc gcg gcc gcc gcc ggt tcg cat cgc aag ttc ctg tac gac acc aac      1824
Arg Ala Ala Ala Ala Gly Ser His Arg Lys Phe Leu Tyr Asp Thr Asn
        595                 600                 605

gtc ggt gcc ggt ctg ccg gtg att gag aac ctg caa aac ctg ctg aac      1872
Val Gly Ala Gly Leu Pro Val Ile Glu Asn Leu Gln Asn Leu Leu Asn
    610                 615                 620

gcc ggt gat gaa ctg gta cgt ttc tcc ggt atc ctg tcc ggc tcg ctg      1920
Ala Gly Asp Glu Leu Val Arg Phe Ser Gly Ile Leu Ser Gly Ser Leu
625                 630                 635                 640

tcc ttt atc ttc ggc aag ctg gac gaa ggg ctg tcg ctg tcg gcg gcg      1968
Ser Phe Ile Phe Gly Lys Leu Asp Glu Gly Leu Ser Leu Ser Ala Ala
                645                 650                 655

acc ctg cag gcc cgg gcc aac ggc tac acc gag ccg gat ccg cgc gac      2016
Thr Leu Gln Ala Arg Ala Asn Gly Tyr Thr Glu Pro Asp Pro Arg Asp
            660                 665                 670

gat ctg tcc ggg atg gat gtg gcg cgt aag ctg ctg atc ctg gcg cgc      2064
Asp Leu Ser Gly Met Asp Val Ala Arg Lys Leu Leu Ile Leu Ala Arg
        675                 680                 685

gag gcc ggt tac aaa ctg gaa ctg agc gat atc gag gtc gag ccg gtg      2112
Glu Ala Gly Tyr Lys Leu Glu Leu Ser Asp Ile Glu Val Glu Pro Val
    690                 695                 700

ctg ccg ccg tcc ttc gac gcg tcg ggc gac gtg gac acc ttc ctg gcg      2160
Leu Pro Pro Ser Phe Asp Ala Ser Gly Asp Val Asp Thr Phe Leu Ala

705                 710                 715                 720

cgg ttg ccg gag ctg gat aaa gag ttc gcg cgc aac gtg gct aac gcc      2208
Arg Leu Pro Glu Leu Asp Lys Glu Phe Ala Arg Asn Val Ala Asn Ala
                725                 730                 735

gcc gag caa ggc aag gtg ctg cgc tat gtc ggc ctg atc gac gaa ggg      2256
Ala Glu Gln Gly Lys Val Leu Arg Tyr Val Gly Leu Ile Asp Glu Gly
            740                 745                 750

cgc tgc aag gtg cgc att gag gcg gtg gac ggc aac gat ccg ttg tat      2304
Arg Cys Lys Val Arg Ile Glu Ala Val Asp Gly Asn Asp Pro Leu Tyr
        755                 760                 765

aaa gtg aag aac ggc gag aac gcg ctg gcc ttc tac agc cgc tac tat      2352
Lys Val Lys Asn Gly Glu Asn Ala Leu Ala Phe Tyr Ser Arg Tyr Tyr
    770                 775                 780

cag ccg ttg ccg ctg gtg ctg cgc ggc tac ggc gcc ggt aac gat gtg      2400
Gln Pro Leu Pro Leu Val Leu Arg Gly Tyr Gly Ala Gly Asn Asp Val
785                 790                 795                 800

act gca gcg ggc gta ttc gcc gat ctg ctg cgc aca ctg tca tgg aag      2448
Thr Ala Ala Gly Val Phe Ala Asp Leu Leu Arg Thr Leu Ser Trp Lys
                805                 810                 815

ttg gga gtt taa                                                      2460
Leu Gly Val

<210>  10
<211>  819
<212>  PRT
<213>  Serratia marcescens

<400>  10

Met Arg Val Leu Lys Phe Gly Gly Thr Ser Val Ala Asn Ala Glu Arg
1               5                   10                  15

Phe Leu Arg Val Ala Asp Ile Met Glu Ser Asn Ala Arg Gln Gly Gln
            20                  25                  30

Val Ala Thr Val Leu Ser Ala Pro Ala Lys Ile Thr Asn His Leu Val
        35                  40                  45

Ala Met Ile Asp Lys Thr Val Ala Gly Gln Asp Ile Leu Pro Asn Met
    50                  55                  60

Ser Asp Ala Glu Arg Ile Phe Ala Asp Leu Leu Ser Gly Leu Ala Gln
65                  70                  75                  80

Ala Leu Pro Gly Phe Glu Tyr Asp Arg Leu Lys Gly Val Val Asp Gln
                85                  90                  95

Glu Phe Ala Gln Leu Lys Gln Val Leu His Gly Val Ser Leu Leu Gly
            100                 105                 110

Gln Cys Pro Asp Ser Val Asn Ala Ala Ile Ile Cys Arg Gly Glu Lys
        115                 120                 125

Leu Ser Ile Ala Ile Met Glu Gly Val Phe Arg Ala Lys Gly Tyr Pro
    130                 135                 140

Val Thr Val Ile Asn Pro Val Glu Lys Leu Leu Ala Gln Gly His Tyr
145                 150                 155                 160

Leu Glu Ser Thr Val Asp Ile Ala Glu Ser Thr Leu Arg Ile Ala Ala
                165                 170                 175

Ala Ala Ile Pro Ala Asp His Ile Val Leu Met Ala Gly Phe Thr Ala
            180                 185                 190

Gly Asn Asp Lys Gly Glu Leu Val Val Leu Gly Arg Asn Gly Ser Asp
        195                 200                 205

Tyr Ser Ala Ala Val Leu Ala Ala Cys Leu Arg Ala Asp Cys Cys Glu
    210                 215                 220

Ile Trp Thr Asp Val Asp Gly Val Tyr Thr Cys Asp Pro Arg Thr Val
225                 230                 235                 240

Pro Asp Ala Arg Leu Leu Lys Ser Met Ser Tyr Gln Glu Ala Met Glu
                245                 250                 255

Leu Ser Tyr Phe Gly Ala Lys Val Leu His Pro Arg Thr Ile Thr Pro
            260                 265                 270

Ile Ala Gln Phe Gln Ile Pro Cys Leu Ile Lys Asn Thr Ser Asn Pro
        275                 280                 285

Gln Ala Pro Gly Thr Leu Ile Gly Lys Asp Ser Thr Asp Ala Asp Met
    290                 295                 300

Pro Val Lys Gly Ile Thr Asn Leu Asn Asn Met Ala Met Ile Asn Val
305                 310                 315                 320

Ser Gly Pro Gly Met Lys Gly Met Val Gly Met Ala Ala Arg Val Phe
                325                 330                 335

Ala Val Met Ser Arg Ala Gly Ile Ser Val Val Leu Ile Thr Gln Ser
            340                 345                 350

Ser Ser Glu Tyr Ser Ile Ser Phe Cys Val Pro Gln Gly Glu Leu Gln
        355                 360                 365

Arg Ala Arg Arg Ala Leu Glu Glu Glu Phe Tyr Leu Glu Leu Lys Asp
    370                 375                 380

Gly Val Leu Asp Pro Leu Asp Val Met Glu Arg Leu Ala Ile Ile Ser
385                 390                 395                 400

Val Val Gly Asp Gly Met Arg Thr Leu Arg Gly Ile Ser Ala Arg Phe
                405                 410                 415

Phe Ser Ala Leu Ala Arg Ala Asn Ile Asn Ile Val Ala Ile Ala Gln
            420                 425                 430

Gly Ser Ser Glu Arg Ser Ile Ser Val Val Val Ser Asn Asp Ser Ala
        435                 440                 445

Thr Thr Gly Val Arg Val Ser His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Ile Glu Gln Ile Tyr Arg Gln Gln Pro Trp Leu Lys Gln Lys His Ile
                485                 490                 495

Asp Leu Arg Val Cys Gly Ile Ala Asn Ser Arg Val Met Leu Thr Asn
            500                 505                 510

Val His Gly Ile Ala Leu Asp Ser Trp Arg Asp Ala Leu Ala Gly Ala
        515                 520                 525

Gln Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Val Asp Phe Leu Ala Asp Gly Phe His Val Val Thr
                565                 570                 575

Pro Asn Lys Lys Ala Asn Thr Ser Ser Met Asn Tyr Tyr Gln Gln Leu
            580                 585                 590

Arg Ala Ala Ala Ala Gly Ser His Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Val Arg Phe Ser Gly Ile Leu Ser Gly Ser Leu
625                 630                 635                 640

Ser Phe Ile Phe Gly Lys Leu Asp Glu Gly Leu Ser Leu Ser Ala Ala
                645                 650                 655

Thr Leu Gln Ala Arg Ala Asn Gly Tyr Thr Glu Pro Asp Pro Arg Asp
            660                 665                 670

Asp Leu Ser Gly Met Asp Val Ala Arg Lys Leu Leu Ile Leu Ala Arg

675                 680                 685

Glu Ala Gly Tyr Lys Leu Glu Leu Ser Asp Ile Glu Val Glu Pro Val
    690                 695                 700

Leu Pro Pro Ser Phe Asp Ala Ser Gly Asp Val Asp Thr Phe Leu Ala
705                 710                 715                 720

Arg Leu Pro Glu Leu Asp Lys Glu Phe Ala Arg Asn Val Ala Asn Ala
                725                 730                 735

Ala Glu Gln Gly Lys Val Leu Arg Tyr Val Gly Leu Ile Asp Glu Gly
            740                 745                 750

Arg Cys Lys Val Arg Ile Glu Ala Val Asp Gly Asn Asp Pro Leu Tyr
        755                 760                 765

Lys Val Lys Asn Gly Glu Asn Ala Leu Ala Phe Tyr Ser Arg Tyr Tyr
    770                 775                 780

Gln Pro Leu Pro Leu Val Leu Arg Gly Tyr Gly Ala Gly Asn Asp Val
785                 790                 795                 800

Thr Ala Ala Gly Val Phe Ala Asp Leu Leu Arg Thr Leu Ser Trp Lys
                805                 810                 815

Leu Gly Val

<210>  11
<211>  2745
<212>  DNA
<213>  Escherichia coli

<220>
<221>  CDS
<222>  (751)..(1995)
<223>  sstT Kodierregion

<400>  11
gatgctggta ctgattctgg tggaattgag cgacgtgatt ttcgccgtgg atagcattcc      60

ggctatcttc gccgtgacca ctgacccgtt cattgtgctg acctcaaacc tgtttgcgat     120

cctcggcctg cgtgcgatgt atttcctgct ggcgggcgta gcagagcgtt tctcgatgct     180

caaatatggc ctggcggtga ttctggtgtt tatcggtatc aagatgctga ttgtcgactt     240

ctaccatatt ccaatcgccg tctcgctggg cgtggtgttt ggcattctgg tgatgacgtt     300

tattatcaac gcctgggtga attatcggca tgataagcag cggggtggat aatttttaat     360

ctgcctaagc cgtgtaccct gtcattaaca tgagcaccgt tttctccctc tcccttccag     420

ggagagggtc ggggtgaggg taatttttcg caccgatgct ggcctgttcc cctcacccta     480

accctctccc caaacggggc gaggggactg accgagtcct tttttgatgt tgtcatcagt     540

ctggaagccg cacgttggct ttatttttat gtcaaagaaa tgtaaccatt aagtttcaaa 600

atatgacctc tctttaaaat ccagcatttt tcgcttcccg aagctgtaac tttccttata 660

ctcgaccttg caaacacttt gttacatcct gaaagatgcg tcgacagaac gcaccaggga 720

tgtgcgacaa cacaatgaaa ggatcgaaaa atg act acg caa cgt tca ccg ggg 774
Met Thr Thr Gln Arg Ser Pro Gly
1               5

cta ttc cgg cgt ctg gct cat ggc agc ctg gta aaa caa atc ctg gtc 822
Leu Phe Arg Arg Leu Ala His Gly Ser Leu Val Lys Gln Ile Leu Val
    10                  15                  20

ggc ctt gtt ctg ggg att ctt ctg gca tgg atc tca aaa ccc gcg gcg 870
Gly Leu Val Leu Gly Ile Leu Leu Ala Trp Ile Ser Lys Pro Ala Ala
25                  30                  35                  40

gaa gct gtt ggt ctg tta ggt act ttg ttc gtc ggc gca ctg aaa gcc 918
Glu Ala Val Gly Leu Leu Gly Thr Leu Phe Val Gly Ala Leu Lys Ala
                45                  50                  55

gtt gcc ccc atc ctg gtg ttg atg ctg gtg atg gca tct att gct aac 966
Val Ala Pro Ile Leu Val Leu Met Leu Val Met Ala Ser Ile Ala Asn
            60                  65                  70

cac cag cac ggg cag aaa acc aat atc cgc cct att ttg ttc ctc tat 1014
His Gln His Gly Gln Lys Thr Asn Ile Arg Pro Ile Leu Phe Leu Tyr
        75                  80                  85

cta ctg ggc acc ttc tct gct gct ctg gcc gca gta gtc ttc agc ttt 1062
Leu Leu Gly Thr Phe Ser Ala Ala Leu Ala Ala Val Val Phe Ser Phe
    90                  95                  100

gcc ttc cct tct acc ctg cat tta tcc agt agc gcg ggt gat att tcg 1110
Ala Phe Pro Ser Thr Leu His Leu Ser Ser Ser Ala Gly Asp Ile Ser
105                 110                 115                 120

ccg ccg tca ggc att gtc gaa gtg atg cgc ggg ctg gta atg agc atg 1158
Pro Pro Ser Gly Ile Val Glu Val Met Arg Gly Leu Val Met Ser Met
                125                 130                 135

gtt tcc aac ccc atc gac gcg ctg ctg aaa ggt aac tac atc ggg att 1206
Val Ser Asn Pro Ile Asp Ala Leu Leu Lys Gly Asn Tyr Ile Gly Ile
            140                 145                 150

ctg gtg tgg gcg atc ggc ctc ggc ttc gca ctg cgt cac ggt aac gag 1254
Leu Val Trp Ala Ile Gly Leu Gly Phe Ala Leu Arg His Gly Asn Glu
        155                 160                 165

acc acc aaa aac ctg gtt aac gat atg tcg aat gcc gtt acc ttt atg 1302
Thr Thr Lys Asn Leu Val Asn Asp Met Ser Asn Ala Val Thr Phe Met
    170                 175                 180

gtg aaa ctg gtc att cgc ttc gca ccg att ggt att ttt ggg ctg gtt 1350
Val Lys Leu Val Ile Arg Phe Ala Pro Ile Gly Ile Phe Gly Leu Val
185                 190                 195                 200

tct tct acc ctg gca acc acc ggt ttc tcc aca ctg tgg ggc tac gcg 1398
Ser Ser Thr Leu Ala Thr Thr Gly Phe Ser Thr Leu Trp Gly Tyr Ala
                205                 210                 215

caa ctg ctg gtc gtg ctg gtt ggc tgt atg tta ctg gtg gcg ctg gtg 1446
Gln Leu Leu Val Val Leu Val Gly Cys Met Leu Leu Val Ala Leu Val
            220                 225                 230

gtt aac cca ttg ctg gtg tgg tgg aaa att cgt cgt aac ccg ttc ccg 1494
Val Asn Pro Leu Leu Val Trp Trp Lys Ile Arg Arg Asn Pro Phe Pro
        235                 240                 245

ctg gtg ctg ctg tgc ctg cgc gaa agc ggt gtg tat gcc ttc ttc acc     1542
Leu Val Leu Leu Cys Leu Arg Glu Ser Gly Val Tyr Ala Phe Phe Thr
    250                 255                 260

cgc agc tct gca gcg aac att ccg gtg aat atg gcg ctg tgt gaa aag     1590
Arg Ser Ser Ala Ala Asn Ile Pro Val Asn Met Ala Leu Cys Glu Lys
265                 270                 275                 280

ctg aat ctg gat cgc gat acc tat tcc gtt tct att ccg ctg gga gcc     1638
Leu Asn Leu Asp Arg Asp Thr Tyr Ser Val Ser Ile Pro Leu Gly Ala
                285                 290                 295

acc atc aat atg gcg ggc gca gca atc act att acc gtg ttg acg ctg     1686
Thr Ile Asn Met Ala Gly Ala Ala Ile Thr Ile Thr Val Leu Thr Leu
            300                 305                 310

gct gcg gtt aat acg ctg ggt att ccg gtc gat ctg ccc acg gcg ctg     1734
Ala Ala Val Asn Thr Leu Gly Ile Pro Val Asp Leu Pro Thr Ala Leu
        315                 320                 325

ctg ttg agc gtg gtg gct tct ctg tgt gcc tgt ggc gca tcc ggc gtg     1782
Leu Leu Ser Val Val Ala Ser Leu Cys Ala Cys Gly Ala Ser Gly Val
    330                 335                 340

gcg ggg ggg tct ctg ctg ctg atc cca ctg gcc tgt aat atg ttc ggt     1830
Ala Gly Gly Ser Leu Leu Leu Ile Pro Leu Ala Cys Asn Met Phe Gly
345                 350                 355                 360

att tcg aac gat atc gcc atg cag gtg gtt gcc gtc ggc ttt atc atc     1878
Ile Ser Asn Asp Ile Ala Met Gln Val Val Ala Val Gly Phe Ile Ile
                365                 370                 375

ggc gta ttg cag gac tct tgc gaa acc gcg ctg aac tct tca act gac     1926
Gly Val Leu Gln Asp Ser Cys Glu Thr Ala Leu Asn Ser Ser Thr Asp
            380                 385                 390

gtg ctg ttc act gcg gca gct tgc cag gca gaa gac gat cgt ctg gca     1974
Val Leu Phe Thr Ala Ala Ala Cys Gln Ala Glu Asp Asp Arg Leu Ala
        395                 400                 405

aat agc gcc ctg cgt aat taa ttgtttaacc cctttcgtct acggcggaag     2025
Asn Ser Ala Leu Arg Asn
    410

gggttttctc aactttaaac ggatcaattc cccttttctg catccgccag aaacgaatga     2085

tattcaggcc attcataagc agaaaactac cctcaatcat cgtgccgcct atcgaccccg     2145

cccagaagtt gtgaatcacc cagcaacacg ttgaaaacca cattacgcag cgcatggtca     2205

gccctttaca gcagaatagc gcccaggtac tgacaatcgt gccgataacc ggcaatagtt     2265

cgacaggatg atggaacttc gcgaggccaa ttccgccagt cagcacaata aaaatcgcca     2325

ttacccataa gctgcgcgtg cgtaaggtaa tcaatgtacg aatggcatta aggatggcac     2385

tggcaccagc gggataggtg cccagaagaa aaaaatgtac gccaataacg gcgctataga     2445

ccgaaagctg ctttttgaag cgacgttcgt cacgattgaa aaatgttgtg ataccaatca     2505

gaaaggcgat gacacccacg ccctgggcca gccaatacgc ggtcatgata aatccttagc     2565

aggtatggaa aagcaaacgg cgcttcacat tatgaaacgc cgttttttat taacaactca     2625

tttcgacttt atagcgttac gccgcttttg aagatcgcca gttcgcggaa gtcgttacgc     2685

tcgttacagg tttgcttacc gttggcaaac tcaacgatgg tgtcgataaa ttcttccagc     2745

<210>  12
<211>  414
<212>  PRT
<213>  Escherichia coli

<400>  12

Met Thr Thr Gln Arg Ser Pro Gly Leu Phe Arg Arg Leu Ala His Gly
1               5                   10                  15

Ser Leu Val Lys Gln Ile Leu Val Gly Leu Val Leu Gly Ile Leu Leu
            20                  25                  30

Ala Trp Ile Ser Lys Pro Ala Ala Glu Ala Val Gly Leu Leu Gly Thr
        35                  40                  45

Leu Phe Val Gly Ala Leu Lys Ala Val Ala Pro Ile Leu Val Leu Met
    50                  55                  60

Leu Val Met Ala Ser Ile Ala Asn His Gln His Gly Gln Lys Thr Asn
65                  70                  75                  80

Ile Arg Pro Ile Leu Phe Leu Tyr Leu Leu Gly Thr Phe Ser Ala Ala
                85                  90                  95

Leu Ala Ala Val Val Phe Ser Phe Ala Phe Pro Ser Thr Leu His Leu
            100                 105                 110

Ser Ser Ser Ala Gly Asp Ile Ser Pro Pro Ser Gly Ile Val Glu Val
        115                 120                 125

Met Arg Gly Leu Val Met Ser Met Val Ser Asn Pro Ile Asp Ala Leu
    130                 135                 140

Leu Lys Gly Asn Tyr Ile Gly Ile Leu Val Trp Ala Ile Gly Leu Gly
145                 150                 155                 160

Phe Ala Leu Arg His Gly Asn Glu Thr Thr Lys Asn Leu Val Asn Asp
                165                 170                 175

Met Ser Asn Ala Val Thr Phe Met Val Lys Leu Val Ile Arg Phe Ala
            180                 185                 190

Pro Ile Gly Ile Phe Gly Leu Val Ser Ser Thr Leu Ala Thr Thr Gly
        195                 200                 205

Phe Ser Thr Leu Trp Gly Tyr Ala Gln Leu Leu Val Val Leu Val Gly
    210                 215                 220

Cys Met Leu Leu Val Ala Leu Val Val Asn Pro Leu Leu Val Trp Trp
225                 230                 235                 240

Lys Ile Arg Arg Asn Pro Phe Pro Leu Val Leu Leu Cys Leu Arg Glu

245                 250                 255

Ser Gly Val Tyr Ala Phe Phe Thr Arg Ser Ser Ala Ala Asn Ile Pro
            260                 265                 270

Val Asn Met Ala Leu Cys Glu Lys Leu Asn Leu Asp Arg Asp Thr Tyr
        275                 280                 285

Ser Val Ser Ile Pro Leu Gly Ala Thr Ile Asn Met Ala Gly Ala Ala
    290                 295                 300

Ile Thr Ile Thr Val Leu Thr Leu Ala Ala Val Asn Thr Leu Gly Ile
305                 310                 315                 320

Pro Val Asp Leu Pro Thr Ala Leu Leu Leu Ser Val Val Ala Ser Leu
                325                 330                 335

Cys Ala Cys Gly Ala Ser Gly Val Ala Gly Gly Ser Leu Leu Leu Ile
            340                 345                 350

Pro Leu Ala Cys Asn Met Phe Gly Ile Ser Asn Asp Ile Ala Met Gln
        355                 360                 365

Val Val Ala Val Gly Phe Ile Ile Gly Val Leu Gln Asp Ser Cys Glu
    370                 375                 380

Thr Ala Leu Asn Ser Ser Thr Asp Val Leu Phe Thr Ala Ala Ala Cys
385                 390                 395                 400

Gln Ala Glu Asp Asp Arg Leu Ala Asn Ser Ala Leu Arg Asn
                405                 410

<210>  13
<211>  2832
<212>  DNA
<213>  Escherichia coli

<220>
<221>  CDS
<222>  (751)..(2082)
<223>  tdcC Kodierregion

<400>  13
caaggggttt ccctctcctg cgcgatgctg ggtatcgacg gtaaagtggt gatgccaaaa      60

ggtgcgccaa aatccaaagt agcggcaacg tgcgactact ccgcagaagt cgttctgcat     120

ggtgataact tcaacgacac tatcgctaaa gtgagcgaaa ttgtcgaaat ggaaggccgt     180

atttttatcc caccttacga tgatccgaaa gtgattgctg gccagggaac gattggtctg     240

gaaattatgg aagatctcta tgatgtcgat aacgtgattg tgccaattgg tggtggcggt     300

ttaattgctg gtattgcggt ggcaattaaa tctattaacc cgaccattcg tgttattggc     360

gtacagtctg aaaacgttca cggcatggcg gcttctttcc actccggaga aataaccacg     420

caccgaacta ccggcaccct ggcggatggt tgtgatgtct cccgcccggg taatttaact 480

tacgaaatcg ttcgtgaatt agtcgatgac atcgtgctgg tcagcgaaga cgaaatcaga 540

aacagtatga ttgccttaat tcagcgcaat aaagtcgtca ccgaaggcgc aggcgctctg 600

gcatgtgctg cattattaag cggtaaatta gaccaatata ttcaaaacag aaaaaccgtc 660

agtattattt ccggcggcaa tatcgatctt tctcgcgtct ctcaaatcac cggtttcgtt 720

gacgcttaat taattcgttg aggataggat atg agt act tca gat agc att gta 774
Met Ser Thr Ser Asp Ser Ile Val
1               5

tcc agc cag aca aaa caa tcg tcc tgg cgt aaa tca gat acc aca tgg 822
Ser Ser Gln Thr Lys Gln Ser Ser Trp Arg Lys Ser Asp Thr Thr Trp
    10                  15                  20

acg tta ggc ttg ttt ggt acg gca atc ggc gcc ggg gtg ctg ttc ttc 870
Thr Leu Gly Leu Phe Gly Thr Ala Ile Gly Ala Gly Val Leu Phe Phe
25                  30                  35                  40

cct atc cgc gca ggt ttt ggc gga ctg atc ccg att ctt ctg atg ttg 918
Pro Ile Arg Ala Gly Phe Gly Gly Leu Ile Pro Ile Leu Leu Met Leu
                45                  50                  55

gta ttg gca tac ccc atc gcg ttt tat tgc cac cgg gcg ctg gcg cgt 966
Val Leu Ala Tyr Pro Ile Ala Phe Tyr Cys His Arg Ala Leu Ala Arg
            60                  65                  70

ctg tgt ctt tct ggc tct aac cct tcc ggc aac att acg gaa acg gtg 1014
Leu Cys Leu Ser Gly Ser Asn Pro Ser Gly Asn Ile Thr Glu Thr Val
        75                  80                  85

gaa gag cat ttt ggt aaa act ggc ggc gtg gtt atc acg ttc ctg tac 1062
Glu Glu His Phe Gly Lys Thr Gly Gly Val Val Ile Thr Phe Leu Tyr
    90                  95                  100

ttc ttc gcg att tgc cca ctg ctg tgg att tat ggc gtt act att acc 1110
Phe Phe Ala Ile Cys Pro Leu Leu Trp Ile Tyr Gly Val Thr Ile Thr
105                 110                 115                 120

aat acc ttt atg acg ttc tgg gaa aac cag ctc ggc ttt gca ccg ctg 1158
Asn Thr Phe Met Thr Phe Trp Glu Asn Gln Leu Gly Phe Ala Pro Leu
                125                 130                 135

aat cgc ggc ttt gtg gcg ctg ttc ctg ttg ctg ctg atg gct ttc gtc 1206
Asn Arg Gly Phe Val Ala Leu Phe Leu Leu Leu Leu Met Ala Phe Val
            140                 145                 150

atc tgg ttt ggt aag gat ctg atg gtt aaa gtg atg agc tac ctg gta 1254
Ile Trp Phe Gly Lys Asp Leu Met Val Lys Val Met Ser Tyr Leu Val
        155                 160                 165

tgg ccg ttt atc gcc agc ctg gtg ctg att tct ttg tcg ctg atc cct 1302
Trp Pro Phe Ile Ala Ser Leu Val Leu Ile Ser Leu Ser Leu Ile Pro
    170                 175                 180

tac tgg aac tct gca gtt atc gac cag gtt gac ctc ggt tcg ctg tcg 1350
Tyr Trp Asn Ser Ala Val Ile Asp Gln Val Asp Leu Gly Ser Leu Ser
185                 190                 195                 200

tta acc ggt cat gac ggt atc ctg atc act gtc tgg ctg ggg att tcc 1398
Leu Thr Gly His Asp Gly Ile Leu Ile Thr Val Trp Leu Gly Ile Ser
                205                 210                 215

atc atg gtt ttc tcc ttt aac ttc tcg cca atc gtc tct tcc ttc gtg 1446
Ile Met Val Phe Ser Phe Asn Phe Ser Pro Ile Val Ser Ser Phe Val
            220                 225                 230

gtt tct aag cgt gaa gag tat gag aaa gac ttc ggt cgc gac ttc acc     1494
Val Ser Lys Arg Glu Glu Tyr Glu Lys Asp Phe Gly Arg Asp Phe Thr
        235                 240                 245

gaa cgt aaa tgt tcc caa atc att tct cgt gcc agc atg ctg atg gtt     1542
Glu Arg Lys Cys Ser Gln Ile Ile Ser Arg Ala Ser Met Leu Met Val
    250                 255                 260

gca gtg gtg atg ttc ttt gcc ttt agc tgc ctg ttt act ctg tct ccg     1590
Ala Val Val Met Phe Phe Ala Phe Ser Cys Leu Phe Thr Leu Ser Pro
265                 270                 275                 280

gcc aac atg gcg gaa gcc aaa gcg cag aat att cca gtg ctt tct tat     1638
Ala Asn Met Ala Glu Ala Lys Ala Gln Asn Ile Pro Val Leu Ser Tyr
                285                 290                 295

ctg gct aac cac ttt gcg tcc atg acc ggt acc aaa aca acg ttc gcg     1686
Leu Ala Asn His Phe Ala Ser Met Thr Gly Thr Lys Thr Thr Phe Ala
            300                 305                 310

att aca ctg gaa tat gcg gct tcc atc atc gca ctc gtg gct atc ttc     1734
Ile Thr Leu Glu Tyr Ala Ala Ser Ile Ile Ala Leu Val Ala Ile Phe
        315                 320                 325

aaa tct ttc ttc ggt cac tat ctg gga acg ctg gaa ggt ctg aat ggc     1782
Lys Ser Phe Phe Gly His Tyr Leu Gly Thr Leu Glu Gly Leu Asn Gly
    330                 335                 340

ctg gtc ctg aag ttt ggt tat aaa ggc gac aaa act aaa gtg tcg ctg     1830
Leu Val Leu Lys Phe Gly Tyr Lys Gly Asp Lys Thr Lys Val Ser Leu
345                 350                 355                 360

ggt aaa ctg aac act atc agc atg atc ttc atc atg ggc tcc acc tgg     1878
Gly Lys Leu Asn Thr Ile Ser Met Ile Phe Ile Met Gly Ser Thr Trp
                365                 370                 375

gtt gtt gcc tac gcc aac ccg aac atc ctt gac ctg att gaa gcc atg     1926
Val Val Ala Tyr Ala Asn Pro Asn Ile Leu Asp Leu Ile Glu Ala Met
            380                 385                 390

ggc gca ccg att atc gca tcc ctg ctg tgc ctg ttg ccg atg tat gcc     1974
Gly Ala Pro Ile Ile Ala Ser Leu Leu Cys Leu Leu Pro Met Tyr Ala
        395                 400                 405

atc cgt aaa gcg ccg tct ctg gcg aaa tac cgt ggt cgt ctg gat aac     2022
Ile Arg Lys Ala Pro Ser Leu Ala Lys Tyr Arg Gly Arg Leu Asp Asn
    410                 415                 420

gtg ttt gtt acc gtg att ggt ctg ctg acc atc ctg aac atc gta tac     2070
Val Phe Val Thr Val Ile Gly Leu Leu Thr Ile Leu Asn Ile Val Tyr
425                 430                 435                 440

aaa ctg ttt taa tccgtaactc aggatgagaa aagagatgaa tgaatttccg         2122
Lys Leu Phe

gttgttttgg ttattaactg tggttcgtct tcgattaagt tttccgtgct cgatgccagc   2182

gactgtgaag tattaatgtc aggtattgcc gacggtatta actcggaaaa tgcattctta   2242

tccgtaaatg ggggagagcc agcaccgctg gctcaccaca gctacgaagg tgcattgaag   2302

gcaattgcat ttgaactgga aaaacggaat ttaaatgaca gtgtggcctt aattggccac   2362

cgcatcgctc acggcggcag tatttttacc gagtccgcca ttattaccga tgaagtcatt   2422

gataatatcc gtcgcgtttc tccactggca cccctgcata attacgccaa tttaagtggt   2482

attgaatcgg cgcagcaatt atttccgggc gtaactcagg tggcggtatt tgataccagt 2542

ttccaccaga cgatggctcc ggaagcttat ttatacggcc tgccgtggaa atattatgaa 2602

gagttaggtg tacgccgtta tggtttccac ggcacgtcgc accgctatgt ttcccagcgc 2662

gcacattcgc tgctgaatct ggcggaagat gactccggcc tggttgtggc gcatcttggc 2722

aatggcgcgt caatctgcgc ggttcgcaac ggtcagagtg ttgatacctc aatgggaatg 2782

acgccgctgg aaggcttgat gatgggtacc cgcagtggcg atgtcgactt 2832

<210> 14
<211> 443
<212> PRT
<213> Escherichia coli

<400> 14

Met Ser Thr Ser Asp Ser Ile Val Ser Ser Gln Thr Lys Gln Ser Ser
1               5                   10                  15

Trp Arg Lys Ser Asp Thr Thr Trp Thr Leu Gly Leu Phe Gly Thr Ala
            20                  25                  30

Ile Gly Ala Gly Val Leu Phe Phe Pro Ile Arg Ala Gly Phe Gly Gly
        35                  40                  45

Leu Ile Pro Ile Leu Leu Met Leu Val Leu Ala Tyr Pro Ile Ala Phe
    50                  55                  60

Tyr Cys His Arg Ala Leu Ala Arg Leu Cys Leu Ser Gly Ser Asn Pro
65                  70                  75                  80

Ser Gly Asn Ile Thr Glu Thr Val Glu Glu His Phe Gly Lys Thr Gly
                85                  90                  95

Gly Val Val Ile Thr Phe Leu Tyr Phe Phe Ala Ile Cys Pro Leu Leu
            100                 105                 110

Trp Ile Tyr Gly Val Thr Ile Thr Asn Thr Phe Met Thr Phe Trp Glu
        115                 120                 125

Asn Gln Leu Gly Phe Ala Pro Leu Asn Arg Gly Phe Val Ala Leu Phe
    130                 135                 140

Leu Leu Leu Leu Met Ala Phe Val Ile Trp Phe Gly Lys Asp Leu Met
145                 150                 155                 160

Val Lys Val Met Ser Tyr Leu Val Trp Pro Phe Ile Ala Ser Leu Val
                165                 170                 175

Leu Ile Ser Leu Ser Leu Ile Pro Tyr Trp Asn Ser Ala Val Ile Asp
            180                 185                 190

Gln Val Asp Leu Gly Ser Leu Ser Leu Thr Gly His Asp Gly Ile Leu

195                 200                 205

Ile Thr Val Trp Leu Gly Ile Ser Ile Met Val Phe Ser Phe Asn Phe
    210                 215                 220

Ser Pro Ile Val Ser Ser Phe Val Val Ser Lys Arg Glu Glu Tyr Glu
225                 230                 235                 240

Lys Asp Phe Gly Arg Asp Phe Thr Glu Arg Lys Cys Ser Gln Ile Ile
                245                 250                 255

Ser Arg Ala Ser Met Leu Met Val Ala Val Val Met Phe Phe Ala Phe
            260                 265                 270

Ser Cys Leu Phe Thr Leu Ser Pro Ala Asn Met Ala Glu Ala Lys Ala
        275                 280                 285

Gln Asn Ile Pro Val Leu Ser Tyr Leu Ala Asn His Phe Ala Ser Met
    290                 295                 300

Thr Gly Thr Lys Thr Thr Phe Ala Ile Thr Leu Glu Tyr Ala Ala Ser
305                 310                 315                 320

Ile Ile Ala Leu Val Ala Ile Phe Lys Ser Phe Phe Gly His Tyr Leu
                325                 330                 335

Gly Thr Leu Glu Gly Leu Asn Gly Leu Val Leu Lys Phe Gly Tyr Lys
            340                 345                 350

Gly Asp Lys Thr Lys Val Ser Leu Gly Lys Leu Asn Thr Ile Ser Met
        355                 360                 365

Ile Phe Ile Met Gly Ser Thr Trp Val Val Ala Tyr Ala Asn Pro Asn
    370                 375                 380

Ile Leu Asp Leu Ile Glu Ala Met Gly Ala Pro Ile Ile Ala Ser Leu
385                 390                 395                 400

Leu Cys Leu Leu Pro Met Tyr Ala Ile Arg Lys Ala Pro Ser Leu Ala
                405                 410                 415

Lys Tyr Arg Gly Arg Leu Asp Asn Val Phe Val Thr Val Ile Gly Leu
            420                 425                 430

Leu Thr Ile Leu Asn Ile Val Tyr Lys Leu Phe
        435                 440

<210>  15
<211>  2772
<212>  DNA
<213>  Escherichia coli

<220>
<221>  CDS
<222>  (751)..(2022)
<223>  yhjV Kodierregion

<400>  15
tcaaatgcag tagttattca ataccaggat aaaccgtacg ttcgcctgaa cggcggcgac      60

tgggtgcctt acccgcagta agaacgaaaa aggccgcaga gtttcccccc tgcggcctgg     120

tccgggcgca aattgccatt acggcagcct gacgcccgca tgacacgtta cttccgcttg     180

ttcagccagt tcacgatcat actggcaaga atgccagcaa tgaccggagc cgctaaatca     240

tgccagaagg ccatgcccag ctctgcgaac gtcatatagc cgcctgtgtt gtaatgacaa     300

cgtttcgcgg ctattcttga gtggtctaga gtcaagatta gcccccgtgg tgttgtcagg     360

tgcatacctg caacgtgcgg gggttttctc tctccagcaa ccaatgccac cagggataaa     420

gcccccgcaa cattgcgcct caccggaacc tttccggctt gccgctgatt ctacgattgc     480

tttttactgt cagcacccgg caatcttcct cattgcgcga tgccttccag cttaatttct     540

ttatttgcaa tagttgcagt tcatatccgg gctgactcca gactggtagt gaaatgcgca     600

attttgtaga attgcgcctc ctcgcgacct ccccagtgaa tctgttgcct cattttttac     660

atactgcgta ttcgacttct ccacctgttg cgcaagagaa actgggttta ttcatttttg     720

cgaggccgac ttctttctgg acaggacttt atg cag cac aac aca cta tcg aaa      774
                                 Met Gln His Asn Thr Leu Ser Lys
                                 1               5

cac aat cag aaa ttg ccg ttt aca cgc tac gac ttc ggc tgg gtt tta      822
His Asn Gln Lys Leu Pro Phe Thr Arg Tyr Asp Phe Gly Trp Val Leu
    10                  15                  20

tta tgc ata ggc atg gcg att ggt gcc gga acc gtg ctg atg cca gta      870
Leu Cys Ile Gly Met Ala Ile Gly Ala Gly Thr Val Leu Met Pro Val
25                  30                  35                  40

caa att ggc ttg aag gga att tgg gta ttt att acc gca gcg atc att      918
Gln Ile Gly Leu Lys Gly Ile Trp Val Phe Ile Thr Ala Ala Ile Ile
                45                  50                  55

gct tat cct gcc acc tgg gta gtg cag gac att tat tta aaa acc ctt      966
Ala Tyr Pro Ala Thr Trp Val Val Gln Asp Ile Tyr Leu Lys Thr Leu
            60                  65                  70

tct gaa agc gat tcc tgt aat gac tac acc gat att atc agt cat tac     1014
Ser Glu Ser Asp Ser Cys Asn Asp Tyr Thr Asp Ile Ile Ser His Tyr
        75                  80                  85

ctg ggg aag aac tgg gga att ttc ctc ggg gtt atc tac ttt ttg atg     1062
Leu Gly Lys Asn Trp Gly Ile Phe Leu Gly Val Ile Tyr Phe Leu Met
    90                  95                  100

att atc cac ggg att ttt atc tac tct ctc tcc gtg gtt ttc gac agc     1110
Ile Ile His Gly Ile Phe Ile Tyr Ser Leu Ser Val Val Phe Asp Ser
105                 110                 115                 120

gcc tcg tac ctg aaa acc ttc ggt tta acc gat gcc gat ctt tca caa     1158
Ala Ser Tyr Leu Lys Thr Phe Gly Leu Thr Asp Ala Asp Leu Ser Gln
                125                 130                 135

tct cta ctt tat aaa gtc gct att ttc gcc gta ctg gtg gcg att gcg     1206
Ser Leu Leu Tyr Lys Val Ala Ile Phe Ala Val Leu Val Ala Ile Ala
            140                 145                 150

tct ggt ggt gaa cga tta ctg ttt aag att tcc ggg cca atg gtg gtg     1254
Ser Gly Gly Glu Arg Leu Leu Phe Lys Ile Ser Gly Pro Met Val Val
        155                 160                 165

gtc aaa gta ggg att att gtc gtg ttc ggt ttt gcg atg atc ccg cac     1302
Val Lys Val Gly Ile Ile Val Val Phe Gly Phe Ala Met Ile Pro His
    170                 175                 180

tgg aat ttc gcc aat ata acc gcc ttc ccg caa gcc tcc gtc ttt ttc     1350
Trp Asn Phe Ala Asn Ile Thr Ala Phe Pro Gln Ala Ser Val Phe Phe
185                 190                 195                 200

cgc gat gtc ttg ctt acc att cca ttt tgc ttc ttt tct gca gta ttt     1398
Arg Asp Val Leu Leu Thr Ile Pro Phe Cys Phe Phe Ser Ala Val Phe
                205                 210                 215

att cag gta ctt aac cca atg aat att gcc tat cgt aaa cgg gaa gcg     1446
Ile Gln Val Leu Asn Pro Met Asn Ile Ala Tyr Arg Lys Arg Glu Ala
            220                 225                 230

gat aaa gta ctg gca acc cgg ctc gcg ctg cgt acc cac cga att agt     1494
Asp Lys Val Leu Ala Thr Arg Leu Ala Leu Arg Thr His Arg Ile Ser
        235                 240                 245

tat atc acg ctc atc gcg gtg atc ctg ttt ttt gcc ttt tcg ttt acc     1542
Tyr Ile Thr Leu Ile Ala Val Ile Leu Phe Phe Ala Phe Ser Phe Thr
    250                 255                 260

ttc tca att agc cac gaa gaa gcc gtt tct gcc ttt gaa caa aat atc     1590
Phe Ser Ile Ser His Glu Glu Ala Val Ser Ala Phe Glu Gln Asn Ile
265                 270                 275                 280

tca gca ctg gcg ctg gcc gcg cag gtg atc cct ggg cat atc att cat     1638
Ser Ala Leu Ala Leu Ala Ala Gln Val Ile Pro Gly His Ile Ile His
                285                 290                 295

atc acc tct acg gtg ctt aat atc ttt gcc gta ctg acc gca ttc ttt     1686
Ile Thr Ser Thr Val Leu Asn Ile Phe Ala Val Leu Thr Ala Phe Phe
            300                 305                 310

ggc att tat ctc ggt ttc cac gag gcc att aaa ggc att att ctc aat     1734
Gly Ile Tyr Leu Gly Phe His Glu Ala Ile Lys Gly Ile Ile Leu Asn
        315                 320                 325

ctg tta agc cga att att gat acc aag aaa att aac tca cgc gtg ctg     1782
Leu Leu Ser Arg Ile Ile Asp Thr Lys Lys Ile Asn Ser Arg Val Leu
    330                 335                 340

act ctg gcg atc tgc gct ttt atc gtc att acg ttg acg att tgg gtt     1830
Thr Leu Ala Ile Cys Ala Phe Ile Val Ile Thr Leu Thr Ile Trp Val
345                 350                 355                 360

tcg ttt cgt gta tcg gtg ctg gtg ttc ttt cag ttg gga agc ccg tta     1878
Ser Phe Arg Val Ser Val Leu Val Phe Phe Gln Leu Gly Ser Pro Leu
                365                 370                 375

tat ggt att gtg tcg tgc ctc att ccg ttt ttc ctg atc tat aaa gtc     1926
Tyr Gly Ile Val Ser Cys Leu Ile Pro Phe Phe Leu Ile Tyr Lys Val
            380                 385                 390

gca caa ctg gaa aaa ctt cgc gga ttt aaa gcc tgg ctg att ctg ctg     1974
Ala Gln Leu Glu Lys Leu Arg Gly Phe Lys Ala Trp Leu Ile Leu Leu
        395                 400                 405

tac ggc att ttg cta tgc ttg tcg cca ctg ttg aag ctg att gag taa     2022
Tyr Gly Ile Leu Leu Cys Leu Ser Pro Leu Leu Lys Leu Ile Glu
    410                 415                 420

accggagcgc atggccccgg ttttgtgagt taacgctgcg gattttcatc ctgatcaaca 2082

gcaaaacaag ctaccagttg accgccgtag tcttttagct gcggctgcaa ctgggtgcag 2142

gggccgaagc gccgacgaca gcgggcgttg aaggcgcaac ccggcggtgg attcagtggg 2202

cttggtagtt caccgctgag cttgatgcgc tcgcggcgat cgtccgggtt caggcgcggc 2262

gtcgcggaaa gtagcgcctg agtgtacgga tggcgcgggt tattgaagat ttggtctttc 2322

gttcccttct ccacgcagcg gcccaggtac atcaccatca cttcatcagc aatgtgctcc 2382

accaccgaca ggtcgtggga gataaagaca taagacagcc ccaactcctg ctgcaaatcc 2442

atcatcagat tcagcacctg cgcgcgcact gaaacatcca gcgcggaaac cggttcatcg 2502

gcaatcacca catccgggtc gagcatcaga ccacgggcga tggcgatacg ctgacgctga 2562

ccgccggaga acatatgcgg atagcggtca tagtgctcgg ttttcaggcc gactttcgcc 2622

atcatcgaca gggctttttc ccgacgctgt tctttgctta agctggtgtt gatcagcagc 2682

ggctcttcaa gaatttgccc gactttttta cgcggattca gcgaaccgta agggttctgg 2742

aagacgatct ggattttctg ccgacgcagc 2772

<210> 16
<211> 423
<212> PRT
<213> Escherichia coli

<400> 16

Met Gln His Asn Thr Leu Ser Lys His Asn Gln Lys Leu Pro Phe Thr
1               5                   10                  15

Arg Tyr Asp Phe Gly Trp Val Leu Leu Cys Ile Gly Met Ala Ile Gly
            20                  25                  30

Ala Gly Thr Val Leu Met Pro Val Gln Ile Gly Leu Lys Gly Ile Trp
        35                  40                  45

Val Phe Ile Thr Ala Ala Ile Ile Ala Tyr Pro Ala Thr Trp Val Val
    50                  55                  60

Gln Asp Ile Tyr Leu Lys Thr Leu Ser Glu Ser Asp Ser Cys Asn Asp
65                  70                  75                  80

Tyr Thr Asp Ile Ile Ser His Tyr Leu Gly Lys Asn Trp Gly Ile Phe
                85                  90                  95

Leu Gly Val Ile Tyr Phe Leu Met Ile Ile His Gly Ile Phe Ile Tyr
            100                 105                 110

Ser Leu Ser Val Val Phe Asp Ser Ala Ser Tyr Leu Lys Thr Phe Gly
        115                 120                 125

Leu Thr Asp Ala Asp Leu Ser Gln Ser Leu Leu Tyr Lys Val Ala Ile
    130                 135                 140

Phe Ala Val Leu Val Ala Ile Ala Ser Gly Gly Glu Arg Leu Leu Phe
145                 150                 155                 160

Lys Ile Ser Gly Pro Met Val Val Val Lys Val Gly Ile Ile Val Val
                165                 170                 175

Phe Gly Phe Ala Met Ile Pro His Trp Asn Phe Ala Asn Ile Thr Ala
            180                 185                 190

Phe Pro Gln Ala Ser Val Phe Phe Arg Asp Val Leu Leu Thr Ile Pro
        195                 200                 205

Phe Cys Phe Phe Ser Ala Val Phe Ile Gln Val Leu Asn Pro Met Asn
    210                 215                 220

Ile Ala Tyr Arg Lys Arg Glu Ala Asp Lys Val Leu Ala Thr Arg Leu
225                 230                 235                 240

Ala Leu Arg Thr His Arg Ile Ser Tyr Ile Thr Leu Ile Ala Val Ile
                245                 250                 255

Leu Phe Phe Ala Phe Ser Phe Thr Phe Ser Ile Ser His Glu Glu Ala
            260                 265                 270

Val Ser Ala Phe Glu Gln Asn Ile Ser Ala Leu Ala Leu Ala Ala Gln
        275                 280                 285

Val Ile Pro Gly His Ile Ile His Ile Thr Ser Thr Val Leu Asn Ile
    290                 295                 300

Phe Ala Val Leu Thr Ala Phe Phe Gly Ile Tyr Leu Gly Phe His Glu
305                 310                 315                 320

Ala Ile Lys Gly Ile Ile Leu Asn Leu Leu Ser Arg Ile Ile Asp Thr
                325                 330                 335

Lys Lys Ile Asn Ser Arg Val Leu Thr Leu Ala Ile Cys Ala Phe Ile
            340                 345                 350

Val Ile Thr Leu Thr Ile Trp Val Ser Phe Arg Val Ser Val Leu Val
        355                 360                 365

Phe Phe Gln Leu Gly Ser Pro Leu Tyr Gly Ile Val Ser Cys Leu Ile
    370                 375                 380

Pro Phe Phe Leu Ile Tyr Lys Val Ala Gln Leu Glu Lys Leu Arg Gly
385                 390                 395                 400

Phe Lys Ala Trp Leu Ile Leu Leu Tyr Gly Ile Leu Leu Cys Leu Ser
                405                 410                 415

Pro Leu Leu Lys Leu Ile Glu
            420

<210>  17
<211>  2730
<212>  DNA
<213>  Escherichia coli

<220>
<221>  CDS
<222>  (751)..(1980)
<223>  yqeG Kodierregion

<400>  17
gagccagatt ttcggcggtg acaccaatat gataatcatt gaaggcatcc cacaacccat      60

catgcacaag actgtcaacc aactggctat tgccaagctg tgcaccggtg cggctatcag     120

tcagaacatg tggtgcgcgg ctcatgtttt cctggccacc ggcgatgaca atatcagcct     180

cgccacactg tatcgcctga gtagccagat gcagtgcttt aagcccggaa ccgcaaacgt     240

cattaatagt gattgcagaa acgctattag gcagaccacc tttaatagcc gattgccttg     300

ccggattctg ccctgcccct gcagtcaaca cctgaccaag aattacttca tccaccgcat     360

atgcaggaac gccggtacgt tctattaacg ctttcacgac cagactacca agttccacgg     420

cggaatgacc cgctaacgca ccacgaaagc agccgatagg tgtccgtaac gccccgacaa     480

tcacaacgtc tttcatcaca acctcgcacc aattaacagt gcaatagtaa atgattgtta     540

attacagtta tcttaattgt ttaaaaaaag tgatttttat cacaaaggaa atatgcctga     600

gcagcagtca gagacataac tggcacgtaa ggtttgcaac cactaaccca ccaatagagg     660

ggtagatagg gcgttaatct cccatactta acctggttta tggtaaattg ccctccattt     720

tgtttaattt gtagatgata cgttcagata atg tct aat att tgg tct aaa gaa      774
                                 Met Ser Asn Ile Trp Ser Lys Glu
                                 1               5

gaa act ctg tgg agt ttc gcg ctc tac ggc aca gcc gtt ggt gca ggc      822
Glu Thr Leu Trp Ser Phe Ala Leu Tyr Gly Thr Ala Val Gly Ala Gly
    10                  15                  20

acg ctc ttc ctt cct att cag tta ggt tcg gca ggg gct gtg gtc ctg      870
Thr Leu Phe Leu Pro Ile Gln Leu Gly Ser Ala Gly Ala Val Val Leu
25                  30                  35                  40

ttt att act gct ctg gtc gcc tgg cct tta aca tat tgg cca cat aaa      918
Phe Ile Thr Ala Leu Val Ala Trp Pro Leu Thr Tyr Trp Pro His Lys
                45                  50                  55

gcc ttg tgc cag ttc atc ctc tca tca aaa aca tca gca ggt gaa ggg      966
Ala Leu Cys Gln Phe Ile Leu Ser Ser Lys Thr Ser Ala Gly Glu Gly
            60                  65                  70

ata acg ggc gcg gta aca cac tac tat ggc aag aag att ggt aat ctg     1014
Ile Thr Gly Ala Val Thr His Tyr Tyr Gly Lys Lys Ile Gly Asn Leu
        75                  80                  85

att acc acg ctg tac ttc atc gcc ttt ttt gtc gtc gtg ttg ata tat     1062
Ile Thr Thr Leu Tyr Phe Ile Ala Phe Phe Val Val Val Leu Ile Tyr
    90                  95                  100

gca gtg gca att acc aac tca ctt acg gaa cag ctg gca aag cat atg     1110

Ala Val Ala Ile Thr Asn Ser Leu Thr Glu Gln Leu Ala Lys His Met
105                 110                 115                 120

gtt att gat ctt cgc atc cgt atg ttg gtg agt ctg ggt gtt gta tta      1158
Val Ile Asp Leu Arg Ile Arg Met Leu Val Ser Leu Gly Val Val Leu
                125                 130                 135

att ctg aat ctc att ttt ctg atg gga cgt cat gcc act att cgg gta      1206
Ile Leu Asn Leu Ile Phe Leu Met Gly Arg His Ala Thr Ile Arg Val
            140                 145                 150

atg gga ttt ttg gta ttc cca ttg att gcc tat ttc tta ttt ctt tcc      1254
Met Gly Phe Leu Val Phe Pro Leu Ile Ala Tyr Phe Leu Phe Leu Ser
        155                 160                 165

att tac ctt gtc ggt agt tgg caa cct gat cta tta aca acc cag gta      1302
Ile Tyr Leu Val Gly Ser Trp Gln Pro Asp Leu Leu Thr Thr Gln Val
    170                 175                 180

gag ttc aat cag aat acc ctt cac cag ata tgg ata tcg att ccc gtg      1350
Glu Phe Asn Gln Asn Thr Leu His Gln Ile Trp Ile Ser Ile Pro Val
185                 190                 195                 200

atg gtt ttc gcc ttt agc cat acg ccc att att tct acg ttt gcc ata      1398
Met Val Phe Ala Phe Ser His Thr Pro Ile Ile Ser Thr Phe Ala Ile
                205                 210                 215

gac aga cgt gaa aaa tat ggc gaa cac gct atg gat aaa tgc aaa aaa      1446
Asp Arg Arg Glu Lys Tyr Gly Glu His Ala Met Asp Lys Cys Lys Lys
            220                 225                 230

att atg aaa gtc gct tat ctc atc atc tgc ata agt gta ctg ttc ttt      1494
Ile Met Lys Val Ala Tyr Leu Ile Ile Cys Ile Ser Val Leu Phe Phe
        235                 240                 245

gtc ttt agc tgc ctg ctt tct att cca cct tcg tat att gaa gct gct      1542
Val Phe Ser Cys Leu Leu Ser Ile Pro Pro Ser Tyr Ile Glu Ala Ala
    250                 255                 260

aaa gaa gaa ggg gtc acc att tta tcg gcg ctt tct atg ctg ccg aac      1590
Lys Glu Glu Gly Val Thr Ile Leu Ser Ala Leu Ser Met Leu Pro Asn
265                 270                 275                 280

gcc cca gca tgg ttg tca att tcc ggg att att gtc gca gta gtt gcg      1638
Ala Pro Ala Trp Leu Ser Ile Ser Gly Ile Ile Val Ala Val Val Ala
                285                 290                 295

atg tcg aaa tca ttc ctg ggt acg tac ttt ggc gtt att gaa ggt gcc      1686
Met Ser Lys Ser Phe Leu Gly Thr Tyr Phe Gly Val Ile Glu Gly Ala
            300                 305                 310

aca gag gtc gtc aaa aca aca cta cag cag gtt ggt gta aag aaa agt      1734
Thr Glu Val Val Lys Thr Thr Leu Gln Gln Val Gly Val Lys Lys Ser
        315                 320                 325

cgt gca ttt aac cgc gca cta tca att atg ttg gta tcg ctg att acc      1782
Arg Ala Phe Asn Arg Ala Leu Ser Ile Met Leu Val Ser Leu Ile Thr
    330                 335                 340

ttc att gtt tgt tgc att aac ccg aac gcg att tcg atg att tac gcg      1830
Phe Ile Val Cys Cys Ile Asn Pro Asn Ala Ile Ser Met Ile Tyr Ala
345                 350                 355                 360

atc agc ggc ccg ctc att gcc atg ata ctt ttc atc atg cct acg ctg      1878
Ile Ser Gly Pro Leu Ile Ala Met Ile Leu Phe Ile Met Pro Thr Leu
                365                 370                 375

tca acg tat ctc atc ccg gcg ctt aaa ccc tgg cgt tcc atc gga aat      1926
Ser Thr Tyr Leu Ile Pro Ala Leu Lys Pro Trp Arg Ser Ile Gly Asn

380                 385                 390

ctg att acg ctg atc gtg ggt atc ctg tgc gta tcg gta atg ttc ttt     1974
Leu Ile Thr Leu Ile Val Gly Ile Leu Cys Val Ser Val Met Phe Phe
        395                 400                 405

agc taa tatcagttgt ggatggttca aaattgctac aattttgaac cattccattt     2030
Ser

ttcataatat tcatttgtta ttattccacc ctatcacttc tctcgtcata tcctagtatt   2090

atcagttagt taaacacaca aagagatctg aacatcttaa ataagactat ttaagatgca   2150

taacttagat tcgcaagata tcttctggtc attcaaaaac aatttgcgat atagatattc   2210

ttataccatg agatagttat ctggaccgct ctcaaccatg caagattaaa tcagtgaaac   2270

ataatcatat ttgataacga gaaatgcatt tttaaatgcc ttactcagaa ttatagcaaa   2330

tacagattaa tcgcaatttt tcccggcaaa attacaaaat catggtgtat tgacatcatc   2390

aataaccaat gaaattatgc aattatatac ggatagggag gttcttaaca tggggctttg   2450

tagtcgttat aaaagtctta catgcaatag ttgctcaatg cattgccaaa taatgccaga   2510

agagtcaccg cgtttgcagt attgcgcaaa ctcgtgtttt tgtatgtggc ccgaagaaag   2570

ttcatatttt aatcgtggtg tggtagaagg tatcttaaca aaaaaccaca atgcgagatt   2630

aagcggatat atttttgtcg atttttcagt aagtttttta cgtctattcc ttgaaaaaga   2690

ctggattgac tatcttgcaa gtactgacat ggggattgtt                         2730

<210>  18
<211>  409
<212>  PRT
<213>  Escherichia coli

<400>  18

Met Ser Asn Ile Trp Ser Lys Glu Glu Thr Leu Trp Ser Phe Ala Leu
1               5                   10                  15

Tyr Gly Thr Ala Val Gly Ala Gly Thr Leu Phe Leu Pro Ile Gln Leu
            20                  25                  30

Gly Ser Ala Gly Ala Val Val Leu Phe Ile Thr Ala Leu Val Ala Trp
        35                  40                  45

Pro Leu Thr Tyr Trp Pro His Lys Ala Leu Cys Gln Phe Ile Leu Ser
    50                  55                  60

Ser Lys Thr Ser Ala Gly Glu Gly Ile Thr Gly Ala Val Thr His Tyr
65                  70                  75                  80

Tyr Gly Lys Lys Ile Gly Asn Leu Ile Thr Thr Leu Tyr Phe Ile Ala
                85                  90                  95

Phe Phe Val Val Val Leu Ile Tyr Ala Val Ala Ile Thr Asn Ser Leu
            100                 105                 110

Thr Glu Gln Leu Ala Lys His Met Val Ile Asp Leu Arg Ile Arg Met
        115                 120                 125

Leu Val Ser Leu Gly Val Val Leu Ile Leu Asn Leu Ile Phe Leu Met
    130                 135                 140

Gly Arg His Ala Thr Ile Arg Val Met Gly Phe Leu Val Phe Pro Leu
145                 150                 155                 160

Ile Ala Tyr Phe Leu Phe Leu Ser Ile Tyr Leu Val Gly Ser Trp Gln
                165                 170                 175

Pro Asp Leu Leu Thr Thr Gln Val Glu Phe Asn Gln Asn Thr Leu His
            180                 185                 190

Gln Ile Trp Ile Ser Ile Pro Val Met Val Phe Ala Phe Ser His Thr
        195                 200                 205

Pro Ile Ile Ser Thr Phe Ala Ile Asp Arg Arg Glu Lys Tyr Gly Glu
    210                 215                 220

His Ala Met Asp Lys Cys Lys Lys Ile Met Lys Val Ala Tyr Leu Ile
225                 230                 235                 240

Ile Cys Ile Ser Val Leu Phe Phe Val Phe Ser Cys Leu Leu Ser Ile
                245                 250                 255

Pro Pro Ser Tyr Ile Glu Ala Ala Lys Glu Glu Gly Val Thr Ile Leu
            260                 265                 270

Ser Ala Leu Ser Met Leu Pro Asn Ala Pro Ala Trp Leu Ser Ile Ser
        275                 280                 285

Gly Ile Ile Val Ala Val Val Ala Met Ser Lys Ser Phe Leu Gly Thr
    290                 295                 300

Tyr Phe Gly Val Ile Glu Gly Ala Thr Glu Val Val Lys Thr Thr Leu
305                 310                 315                 320

Gln Gln Val Gly Val Lys Lys Ser Arg Ala Phe Asn Arg Ala Leu Ser
                325                 330                 335

Ile Met Leu Val Ser Leu Ile Thr Phe Ile Val Cys Cys Ile Asn Pro
            340                 345                 350

Asn Ala Ile Ser Met Ile Tyr Ala Ile Ser Gly Pro Leu Ile Ala Met
        355                 360                 365

Ile Leu Phe Ile Met Pro Thr Leu Ser Thr Tyr Leu Ile Pro Ala Leu
    370                 375                 380

Lys Pro Trp Arg Ser Ile Gly Asn Leu Ile Thr Leu Ile Val Gly Ile
385                 390                 395                 400

Leu Cys Val Ser Val Met Phe Phe Ser
                405